# EUROPEAN PATENT APPLICATION

(11) **EP 4 328 586 A1**
(43) Date of publication of application: **28.02.2024**
(21) Application number: 23737288.3
(22) Date of filing: 05.01.2023
(51) Int. Cl.: G01N 33/48, C07D 311/82, G01N 33/15, G01N 33/50

(54) **FLUORESCENT STAINING METHOD**

(30) Priority: 06.01.2022 JP 2022001268
(71) Applicant: OSAKA UNIVERSITY, Suita-shi Osaka 565-0871 (JP); Goryo Chemical, Inc., Sapporo-shi, Hokkaido 060-0008 (JP)
(72) Inventor: NOJIMA Satoshi, Suita-shi, Osaka 565-0871 (JP); MORII Eiichi, Suita-shi, Osaka 565-0871 (JP); SUZUKI Yuki, Sapporo-shi, Hokkaido 060-0008 (JP); MINOURA Itsushi, Sapporo-shi, Hokkaido 060-0008 (JP)
(74) Representative: Müller-Boré & Partner Patentanwälte PartG mbB
(86) International application number: PCT/JP2023/000031
(87) International publication number: WO 2023/132337

(57) **Abstract**

The present invention develops a fluorescent staining method compatible with PAS staining that is special staining including staining a structure (e.g., mucus or a basement membrane) containing a large amount of a substance containing a glycol group such as a polysaccharide, the fluorescent staining method enabling clear three-dimensional observation. The present invention provides a method of fluorescently staining a substance containing a glycol group in a tissue or cell sample, the method including bringing a compound represented by the following general formula (I) or a salt thereof and the tissue or cell sample into contact with each other.

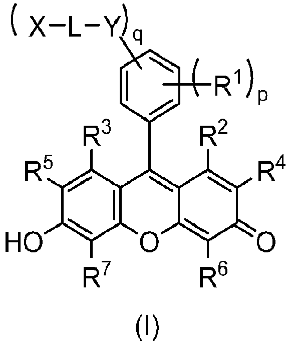

## Description

### Technical Field

The present invention relates to a fluorescent staining method for specifically staining a substance containing a glycol group in a tissue or a cell.

### Background Art

Histopathological diagnosis is a medical practice including microscopically diagnosing the histological morphological change of a lesion site, and is an important diagnostic technology affecting the basis of the decision of a treatment policy for a patient. The histopathological diagnosis is performed as follows: a trained pathologist microscopically examines a glass slide produced by staining a sliced tissue through hematoxylin eosin staining (HE staining). To perform more accurate diagnosis, observation in which an approach to staining a specific structure in a tissue, such as PAS staining, PAM staining, or Alcian Blue staining that is called special staining, is used in combination with the above-mentioned staining is effective in the definite diagnosis of many diseases.

Meanwhile, in the field of basic life sciences, various cutting-edge imaging technologies have been achieving remarkable development in recent years. In particular, a three-dimensional imaging approach that is not achieved until a tissue is cleared is a technology that has been attracting large attention in recent years, and one typical example thereof is a clear, unobstructed brain/body imaging cocktails and computational analysis (CUBIC) method. It has been reported that when three-dimensional observation including using the CUBIC method is combined with an existing fluorescent probe or labeled antibody, the stereoscopic construction of a tissue can be observed at a single-cell level resolution (Non Patent Literature 1).

In such cutting-edge three-dimensional imaging method, the whole-mount staining of a tissue with a fluorescent probe or a fluorescent-labeled antibody (an approach including staining a tissue fragment as a whole without performing slicing) is required because an advanced fluorescence microscope serves as a technical basis. Only fluorescent probes corresponding to some structures, such as a nucleus and actin, exist, and a fluorescent probe developed from the viewpoint of sufficiently being adapted to special staining in clinicopathological diagnosis is substantially absent at present. In, for example, Patent Literature 1, there is a proposal of a method including staining a substance having an aldehyde group in a cell in a sliced tissue section. However, clear three-dimensional imaging of the substance is difficult to achieve by the method of Patent Literature 1.

### Citation List

### Patent Literature

[PTL 1] JP 2018-162986 A

### Non Patent Literature

[NPL 1] Satoshi Nojima et al., Scientific Reports (2014) 7: 9269

### Summary of Invention

### Technical Problem

An object of the present invention is to develop a fluorescent staining method compatible with PAS staining that is special staining including staining a structure (e.g., mucus or a basement membrane) containing a large amount of a substance containing a glycol group such as a polysaccharide, the fluorescent staining method enabling clear three-dimensional observation.

### Solution to Problem

The inventors of the present invention have paid attention to the excitation wavelength of the fluorescent probe used in the method of Patent Literature 1, and have predicted that the excitation wavelength is a short wavelength, and hence the tissue permeability of excitation light is low, with the result that clear three-dimensional imaging (3D imaging) becomes difficult. The inventors have attempted fluorescent staining with various compounds having longer excitation wavelengths on the basis of the prediction. As a result, the inventors have found that the use of a compound having a fluorescein skeleton enables special staining compatible with PAS staining, and the combination thereof with a tissue clearing technology and a 3D imaging technology enables clear three-dimensional observation.

That is, according to one aspect of the present invention, there is provided a method of fluorescently staining a substance containing a glycol group in a tissue or cell sample, the method including bringing a compound represented by the following general formula (I) or a salt thereof and the tissue or cell sample into contact with each other: 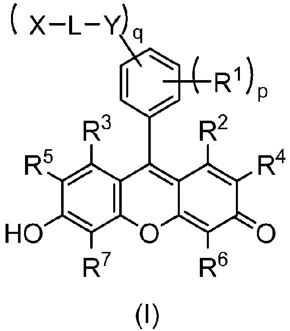 in the general formula (I):
R¹ represents a carboxyl group, an alkyl group that may be substituted, an alkoxy group that may be substituted, or a halogen atom;
R², R³, R⁴, R⁵, R⁶, and R⁷ each independently represent a hydrogen atom, a hydroxyl group (OH group), a halogen atom, or a linear or branched alkyl group that may be substituted;
X represents a hydrazide group, a semicarbazide group, a thiosemicarbazide group, a hydrazino group, a hydroxylamino group, an amino group, or an alkylamino group;
Y is absent or represents an amide group, an ester group, a carbamide group, a thiourea group, a carbamate group, a carbonate group, or an oxygen atom;
L represents -(linear or branched alkylene)ₙ- where "n" represents an integer of from 1 to 10, -(linear or branched alkylene-0-)ₙ- where "n" represents an integer of from 1 to 10, or -(linear or branched alkylene-O-)ₙ-(linear alkylene)ₘ- where "n" and "m" each independently represent an integer of from 1 to 10, or represents a single bond;
"p" represents an integer of from 0 to 4, and when "p" represents 2 or more, R¹s may be identical to or different from each other; and
"q" represents an integer of 1 or 2, and when "q" represents 2, structures each represented by (X-L-Y) may be identical to or different from each other, and a sum of "p" and "q" is an integer of 5 or less.

According to another aspect of the present invention, there is provided a method of detecting a substance containing a glycol group in a tissue or cell sample, the method including: subjecting the substance containing a glycol group in the tissue or cell sample to fluorescent staining by the above-mentioned fluorescent staining method; and measuring fluorescence derived from the above-mentioned compound.

According to another aspect of the present invention, there is provided a method of visualizing a tissue or a cell containing a substance containing a glycol group, the method including: subjecting the substance containing a glycol group in a tissue or cell sample to fluorescent staining by the above-mentioned fluorescent staining method; and measuring and imaging fluorescence derived from the above-mentioned compound.

According to another aspect of the present invention, there is provided a method of determining a tissue and/or a lesion containing a large amount of a substance containing a glycol group in a tissue or cell sample, the method including: subjecting the substance containing a glycol group in the tissue or cell sample to fluorescent staining by the above-mentioned fluorescent staining method; and determining that the tissue and/or the lesion is present in a stained site.

According to another aspect of the present invention, there is provided a method of screening a therapeutic agent for a disease involving a lesion of a tissue or a cell containing a substance containing a glycol group, the method including: administering a therapeutic agent candidate to an individual suffering from the disease; collecting a tissue or cell sample in which the lesion occurs from the individual; subjecting the substance containing a glycol group in the tissue or cell sample to fluorescent staining by the above-mentioned fluorescent staining method; measuring and imaging fluorescence derived from the above-mentioned compound to visualize the tissue or the cell containing the substance containing a glycol group; and determining a therapeutic effect of the therapeutic agent candidate on the basis of an image of the tissue or the cell containing the substance containing a glycol group that has been visualized.

According to another aspect of the present invention, there is provided a composition for fluorescent staining, including a compound represented by the general formula (I) or a salt thereof, wherein the composition is used for visualizing a tissue or a cell containing a substance containing a glycol group.

According to another aspect of the present invention, there is provided a diagnostic composition for a disease involving a lesion of a tissue or a cell containing a substance containing a glycol group, including a compound represented by the general formula (I) or a salt thereof.

### Advantageous Effects of Invention

According to the present invention, the use of the compound represented by the general formula (I) (hereinafter sometimes referred to as "compound (I)") as a stain enables specific fluorescent staining of a substance containing a glycol group. In addition, the combination of the fluorescent staining method of the present invention with a tissue clearing technology and a 3D imaging technology can stain the substance present deep in a tissue to three-dimensionally visualize the tissue, a cell, or a structure containing the substance.

### Brief Description of Drawings

FIGS. **1** are graphs for showing the fluorescence spectra of DAF-2 and Compound 2 before the addition of an aldehyde and after the addition (FIG. **1A** corresponds to DAF-2 and FIG. **1B** corresponds to Compound 2).
FIG. **2** shows photographs obtained by staining a goblet cell of a colonic mucosal epithelium with DAF-2 or Compound 2. The upper portion of the photographs represents a used stain. The result of the staining with each stain, the result of DAPI staining, and a result obtained by merging the results are shown in the stated order from upper photographs.
FIG. **3** shows photographs obtained by staining the basement membrane of a renal glomerulus with DAF-2 or Compound 2. The upper portion of the photographs represents a used stain. The result of the staining with each stain, the result of DAPI staining (a cell nucleus is stained), and a result obtained by merging the results are shown in the stated order from upper photographs.
FIG. **4A** shows the result of the 3D imaging of the colonic mucosal epithelium in which staining with DAF-2 and a CUBIC method are used in combination.
FIG. **4B** shows the result of the 3D imaging of the colonic mucosal epithelium in which staining with Compound 2 and the CUBIC method are used in combination.
FIG. **5** is a set of photographs and a graph for showing results obtained by subjecting a frozen sliced specimen of a human colonic mucosal tissue to fluorescent staining.
FIG. **6** is a set of photographs and a graph for showing results obtained by clearing the human colonic mucosal tissue and subjecting the tissue to fluorescent staining.
FIG. **7** is a set of photographs for showing results obtained by clearing the human colonic mucosal tissue and subjecting the tissue to fluorescent staining with FAM hydrazide.
FIGS. **8** are photographs for showing results obtained by clearing the human colonic mucosal tissue and subjecting the tissue to fluorescent staining with FAM hydrazide.
FIG. **9** is a set of photographs for showing results obtained by clearing the human colonic mucosal tissue and subjecting the tissue to fluorescent staining with FAM hydrazide.
FIG. **10** is a photograph for showing a result obtained by clearing the human colonic mucosal tissue and subjecting the tissue to fluorescent staining with each of FAM hydrazide and Compound 1.
FIG. **11** is a set of photographs for showing results obtained by clearing a colonic tissue analyte collected from a human suffering from ulcerative colitis and subjecting the analyte to fluorescent staining with FAM hydrazide.
FIG. **12** is a set of photographs for showing results obtained by clearing the colonic tissue analyte collected from the human suffering from ulcerative colitis and subjecting the analyte to fluorescent staining with FAM hydrazide.
FIG. **13** is a set of photographs for showing results obtained by clearing the colonic tissue analyte collected from the human suffering from ulcerative colitis and subjecting the analyte to fluorescent staining with FAM hydrazide.
FIG. **14** is a set of photographs for showing a result obtained by clearing a colonic tissue and subjecting the tissue to fluorescent staining with FAM hydrazide, a result obtained by subjecting the tissue to HE staining, and a result obtained by subjecting the tissue to PAS staining.
FIG. **15** is a pair of views for illustrating the ellipticity and tortuosity of a crypt.
FIG. **16** is a set of graphs for showing results obtained by analyzing crypt distortion through use of its volume, ellipticity, and tortuosity as parameters for all cases.
FIG. **17** is a set of graphs for showing results obtained by analyzing the crypt distortion through use of its volume, ellipticity, and tortuosity as parameters for a case diagnosed as non-specific colitis by a pathologist.
FIGS. **18** are a set of photographs, views, and a graph for showing results obtained by subjecting the crypt distortion to three-dimensional morphological observation.
FIG. **19** is a set of photographs for showing results obtained by subjecting the human colonic mucosal tissue to 3D tissue staining.
FIG. **20** is a set of photographs for showing results obtained by subjecting fungal hyphae to 3D tissue staining.

### Description of Embodiments

### A. Fluorescent Staining Method

A fluorescent staining method according to an embodiment of the present invention is a method of subjecting a substance containing a glycol group in a tissue or cell sample to fluorescent staining, the method including bringing a compound (I) or a salt thereof and the tissue or cell sample into contact with each other. The fluorescent staining method according to the embodiment may further include oxidizing a glycol group in the tissue or cell sample to produce an aldehyde group before bringing the compound (I) or the salt thereof and the tissue or cell sample into contact with each other. In addition, the fluorescent staining method according to the embodiment may further include treating the tissue or cell sample with a clearing agent to clear the tissue or cell sample.

### A-1. Compound (I)

The compound (I) is represented by the following general formula (I): 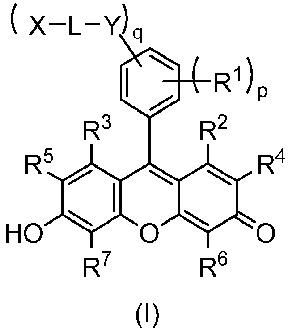 in the general formula (I):
R¹ represents a carboxyl group, an alkyl group that may be substituted, an alkoxy group that may be substituted, or a halogen atom;
R², R³, R⁴, R⁵, R⁶, and R⁷ each independently represent a hydrogen atom, a hydroxyl group (OH group), a halogen atom, or a linear or branched alkyl group that may be substituted;
X represents a hydrazide group, a semicarbazide group, a thiosemicarbazide group, a hydrazino group, a hydroxylamino group, an amino group, or an alkylamino group;
Y is absent or represents an amide group, an ester group, a carbamide group, a thiourea group, a carbamate group, a carbonate group, or an oxygen atom;
L represents -(linear or branched alkylene)ₙ- where "n" represents an integer of from 1 to 10, -(linear or branched alkylene-0-)ₙ- where "n" represents an integer of from 1 to 10, or -(linear or branched alkylene-O-)ₙ-(linear alkylene)ₘ- where "n" and "m" each independently represent an integer of from 1 to 10, or represents a single bond;
"p" represents an integer of from 0 to 4, and when "p" represents 2 or more, R¹s may be identical to or different from each other; and
"q" represents an integer of 1 or 2, and when "q" represents 2, structures each represented by (X-L-Y) may be identical to or different from each other, and a sum of "p" and "q" is an integer of 5 or less.

Herein, the term "alkyl group" or "alkyl" moiety in the other group means a linear or branched saturated hydrocarbon group. The group is preferably a saturated hydrocarbon group having 1 to 6 carbon atoms, and examples thereof include a methyl group, an ethyl group, a n-propyl group, an i-propyl group, a n-butyl group, a sec-butyl group, a t-butyl group, an isobutyl group, a pentyl group, an isopentyl group, a 2,3-dimethylpropyl group, and a hexyl group. The group is more preferably a C1-5 alkyl group, and examples thereof include a methyl group, an ethyl group, a n-propyl group, an i-propyl group, a n-butyl group, a sec-butyl group, a t-butyl group, an isobutyl group, a pentyl group, an isopentyl group, and a 2,3-dimethylpropyl group. The group is still more preferably a C1-3 alkyl group, and examples thereof include a methyl group, an ethyl group, a n-propyl group, and an i-propyl group. The group is most preferably a methyl group or an ethyl group. In addition, the term "alkylene group" refers to a divalent group obtained by removing one hydrogen atom from the above-mentioned alkyl group.

Herein, the term "alkoxy group" refers to a group to be bonded to the above-mentioned alkyl group via an oxygen atom ((alkyl group)-O- group), and the alkyl group moiety is as defined in the foregoing. For example, the number of carbon atoms of the alkyl group moiety in the alkoxy group may be from 1 to 6. Examples of the alkoxy group include a methoxy group, an ethoxy group, a 1-propyloxy group, a 2-propyloxy group, a 2-methyl-1-propyloxy group, a 2-methyl-2-propyloxy group, a 2,2-dimethyl-1-propyloxy group, a 1-butyloxy group, a 2-butyloxy group, a 2-methyl-1-butyloxy group, a 3-methyl-1-butyloxy group, a 2-methyl-2-butyloxy group, a 3-methyl-2-butyloxy group, a 1-pentyloxy group, a 2-pentyloxy group, a 3-pentyloxy group, a 2-methyl-1-pentyloxy group, a 3-methyl-1-pentyloxy group, a 2-methyl-2-pentyloxy group, a 3-methyl-2-pentyloxy group, a 1-hexyloxy group, a 2-hexyloxy group, and a 3-hexyloxy group. The C1-6 alkoxy group is preferably a C1-5 alkoxy group, more preferably a methoxy group, an ethoxy group, a n-propyloxy group, an i-propyloxy group, a n-butyloxy group, a sec-butyloxy group, a t-butyloxy group, an isobutyloxy group, a pentyloxy group, an isopentyloxy group, and a 2,3-dimethylpropyloxy group.

Herein, the term "halogen atom" means a fluorine atom, a chlorine atom, a bromine atom, and an iodine atom, and the halogen atom is preferably a fluorine atom, a chlorine atom, and a bromine atom, more preferably a fluorine atom or a chlorine atom.

Herein, the term "carboxyl group" refers to a group represented by -C(=O)-OH.

Throughout this description, examples of a substituent in the alkyl group or the alkyl moiety of the other group may include a halogen atom, a hydroxyl group, a dialkylamino group, a thiol group, an alkylthiol group, a sulfonyl group, an alkylsulfonyl group, an alkoxy group, a cyclic ether, a carboxyl group, an alkylcarbonyl group, an alkoxycarbonyl group, an alkoxycarbonylamino group, an alkylcarbonyloxy group, an alkylaminocarbonyl group, an alkylcarbonylamino group, and a carbonylamino group. When the alkyl group is substituted, the number of the substituents may be set to, for example, from 1 to 6, from 1 to 5, from 1 to 4, from 1 to 3, 1 or 2, 1, 2, or 3.

R¹ represents preferably a carboxyl group, an alkyl group, or an alkoxy group, more preferably a carboxyl group, a methyl group, or a methoxy group. "p" represents preferably an integer of from 0 to 2, more preferably 1.

R² preferably represents a hydrogen atom.

R³ preferably represents a hydrogen atom.

R⁴ preferably represents a hydrogen atom or a fluorine atom.

R⁵ preferably represents a hydrogen atom or a fluorine atom.

R⁶ preferably represents a hydrogen atom.

R⁷ preferably represents a hydrogen atom.

X represents preferably a hydrazide group, a semicarbazide group, an amino group, or an alkylamino group, more preferably a hydrazide group.

Y is preferably absent or preferably represents an amide group.

L preferably represents a single bond or -(linear or branched alkylene)ₙ- where "n" represents an integer of from 1 to 10.

In the formula (I), for example, a group represented by X-L-Y- may be a group selected from the following where "m" represents an integer of from 1 to 6, and "n" represents an integer of from 1 to 3.

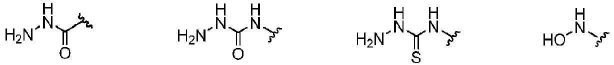

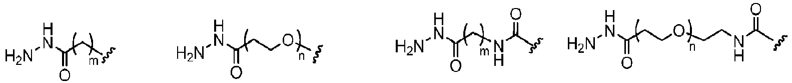

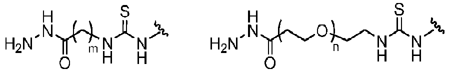

In addition, in the formula (I), for example, a partial structure represented by 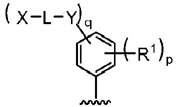 may be a structure represented by any one of the following formulae.

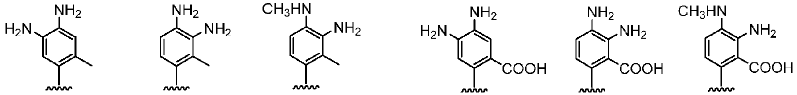

In one embodiment, in the general formula (I):
R¹ represents a carboxyl group, an alkyl group that may be substituted, an alkoxy group that may be substituted, or a halogen atom;
R², R³, R⁶, and R⁷ each independently represent a hydrogen atom, a hydroxyl group (OH group), a halogen atom, or a linear or branched alkyl group that may be substituted;
R⁴ and R⁵ each represent a fluorine atom;
X represents a hydrazide group, a semicarbazide group, a thiosemicarbazide group, a hydrazino group, or a hydroxylamino group;
Y is absent or represents an amide group, an ester group, a carbamide group, a thiourea group, a carbamate group, a carbonate group, or an oxygen atom;
L represents -(linear or branched alkylene)ₙ- where "n" represents an integer of from 1 to 10, -(linear or branched alkylene-O-)ₙ- where "n" represents an integer of from 1 to 10, or -(linear or branched alkylene-O-)ₙ-(linear alkylene)ₘ- where "n" and "m" each independently represent an integer of from 1 to 10, or represents a single bond;
"p" represents an integer of from 0 to 4, and when "p" represents 2 or more, R¹s may be identical to or different from each other; and
"q" represents an integer of 1 or 2, and when "q" represents 2, structures each represented by (X-L-Y) may be identical to or different from each other, and a sum of "p" and "q" is an integer of 5 or less.

In one embodiment, in the general formula (I):
R¹ represents an alkyl group that may be substituted, an alkoxy group that may be substituted, or a halogen atom;
R², R³, R⁴, R⁵, R⁶, and R⁷ each independently represent a hydrogen atom, a hydroxyl group (OH group), a halogen atom, or a linear or branched alkyl group that may be substituted;
X represents an amino group or an alkylamino group;
Y is absent;
L represents a single bond;
"p" represents an integer of from 0 to 4, and when "p" represents 2 or more, R¹s may be identical to or different from each other; and
"q" represents an integer of 1 or 2, and when "q" represents 2, structures each represented by (X-L-Y) may be identical to or different from each other, and a sum of "p" and "q" is an integer of 5 or less.

Specific examples of the compound (I) may include the following compounds.

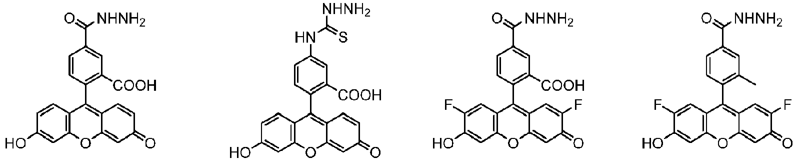

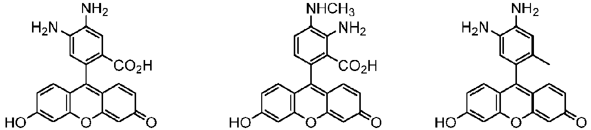

Examples of the salt of the compound (I) may include a base addition salt, an acid addition salt, and an amino acid salt. Examples of the base addition salt may include: metal salts, such as a lithium salt, a sodium salt, a potassium salt, a calcium salt, and a magnesium salt; organic amine salts, such as an ammonium salt, a methylamine salt, a dimethylamine salt, a dicyclohexylamine salt, a tris(hydroxymethyl)aminomethane salt, an N,N-bis(hydroxyethyl)piperazine salt, a 2-amino-2-methyl-1-propanol salt, an ehanolamine salt, an N-methylglucamine salt, an L-glucamine salt, a triethylamine salt, a piperidine salt, and a morpholine salt; and salts with basic amino acids, such as lysine, δ-hydroxylysine, and arginine. Examples of the acid addition salt may include: mineral acid salts, such as a hydrochloric acid salt, a hydrobromic acid salt, a sulfuric acid salt, a nitric acid salt, and a phosphoric acid salt; and organic acid salts, such as a methanesulfonic acid salt, a benzenesulfonic acid salt, a para-toluenesulfonic acid salt, a citric acid salt, an oxalic acid salt, an acetic acid salt, a propionic acid salt, a tartaric acid salt, a fumaric acid salt, a maleic acid salt, a malic acid salt, a succinic acid salt, a benzoic acid salt, a mandelic acid salt, a cinnamic acid salt, a lactic acid salt, a glycolic acid salt, a glucuronic acid salt, an ascorbic acid salt, a nicotinic acid salt, a salicylic acid salt, and a trifluoroacetic acid salt. The amino acid salt may be, for example, a glycine salt. Of course, the salt of the compound of the present invention is not limited thereto.

The compound (I) may have one or two or more asymmetric carbon atoms in accordance with the kind of a substituent thereof, and hence a stereoisomer, such as an optical isomer or a diastereoisomer, may exist. A stereoisomer in a pure form, any appropriate mixture of stereoisomers, a racemic form, and the like are each included in the scope of the present invention. In addition, although the compound represented by the general formula (I) or the salt thereof may exist as a hydrate or a solvate, these substances are each included in the scope of the present invention. Although the kind of a solvent that forms the solvate is not particularly limited, examples thereof may include solvents, such as ethanol, acetone, and isopropanol. The reference "compound (I)" as used herein encompasses any appropriate mixture of the isomers of the compound (I) or a specific stereoisomer thereof, a pharmacologically acceptable salt, hydrate, and solvate of the compound (I), and a hydrate or solvate of a pharmacologically acceptable salt of the compound (I) even when no such compound is clearly described except for the case where such compound is obviously unsuitable. The compound (I) may be synthesized by using a synthesis scheme described in Examples and a synthesis method known in the art.

### A-2. Tissue or Cell Sample

The tissue or cell sample is not particularly limited as long as the sample contains a tissue or a cell of an object for which the presence of the substance containing a glycol group is recognized. The tissue or cell sample is typically derived from a human or a mammal except the human, and a tissue or a cell extracted or excised from the human or the mammal except the human may be used.

The term "tissue" as used herein may refer to the entirety of a tissue or an organ derived from a living organism, or part of the tissue or the organ (e.g., a tissue fragment). Alternatively, the term may mean the entirety of a biont such as an experimental animal itself. In addition, a tissue incorporated into the tissue or cell sample in this description is not required to have a function or a structure as a generally recognized "tissue" and hence also encompasses a cell cluster or a cultured cell that does not have any structure or function expected as a specific tissue. For example, the tissue may be a tissue that may contain a human cancer cell or a tissue fragment excised by carcinectomy.

The term "cell" as used herein refers to general cells derived from living organisms. The term refers to all cell analytes including: an analyte obtained by isolating a cell that has been present in a body fluid or a tissue of a living organism; and a cell artificially produced by subjecting a living organism to specific experimental treatment. Examples of such experimental treatment include such formalin fixation, paraffin embedding, deparaffinization treatment, and tissue clearing treatment as described later. In addition, the cell may be a cell cluster formed by culture, and such cell cluster may be each of a stem cell and a cell cluster obtained by subjecting the stem cell to differentiation induction treatment.

The tissue or cell sample preferably has a three-dimensional stereoscopic structure. The term "three-dimensional stereoscopic structure" means a structure having a thickness in a three-dimensional direction, and the term is used for the purpose of distinguishing the structure from an analyte (sliced section) that has undergone tissue slicing essential at the time of the production of a glass slide standardly used at the time of the performance of histopathological diagnosis. Accordingly, even in the case of a tissue specimen having a plate-shaped or flat structure, when the specimen has such a thickness as to be sufficiently distinguishable from the sliced section (when the specimen has a thickness of, for example, 50 um or more, or, for example, 500 um or more), the specimen is included in the category of a tissue having a three-dimensional stereoscopic structure. Herein, the tissue slicing may be rephrased as a technique to be performed with a microtome. In addition to a tissue fragment and a cell cluster, an organ itself and an individual itself are also included in the category of the tissue having a three-dimensional stereoscopic structure.

The tissue or cell sample may remain as it is after its collection from a living organism, or may be in the state of being subjected to pretreatment. For example, the tissue or cell sample embedded in a paraffin block may be used in the method of the present invention by being subjected to deparaffinization treatment before its staining (see Satoshi Nojima et al., Scientific Reports (2017) 7: 9269).

In one embodiment, the tissue or cell sample is subjected to the following treatment before its contact with the compound (I): a glycol group in the tissue or cell sample is oxidized to produce an aldehyde group. Alternatively, the fluorescent staining method according to the embodiment of the present invention may include oxidizing a glycol group in the tissue or cell sample to produce an aldehyde group before bringing the compound (I) and the tissue or cell sample into contact with each other. This is because the compound (I) is bonded to an aldehyde group to perform staining.

The oxidation treatment of a glycol group has already been well known in a PAS staining method. The oxidation treatment may be typically performed by treating the tissue or cell sample with a 0.5% to 1% aqueous solution of periodic acid for 10 minutes or more, for example, from 10 minutes to 30 minutes.

In one embodiment, the tissue or cell sample is a tissue or cell sample subjected to clearing treatment before its contact with the compound (I). Alternatively, the fluorescent staining method according to the embodiment of the present invention may further include treating the tissue or cell sample with a clearing agent to clear the tissue or cell sample. At this time, the order of the clearing of the tissue or cell sample, and the contact between the compound (I) and the tissue or cell sample is not limited, and the clearing and the contact may be performed at any appropriate timings in accordance with a clearing treatment method or the like. For example, the compound (I) and the tissue or cell sample may be brought into contact with each other before the clearing treatment, simultaneously with the clearing treatment, during the clearing treatment, or after the clearing treatment. The compound (I) can permeate the inside of the tissue or cell sample having a three-dimensional stereoscopic structure to perform staining. Accordingly, when fluorescence derived from the compound (I) is measured after the compound (I) has been caused to permeate the inside of the cleared tissue or cell sample to be bonded to the substance containing a glycol group, the substance containing a glycol group inside the tissue or cell sample having a three-dimensional stereoscopic structure or in the entirety thereof can be detected.

The clearing treatment may be performed by treating the tissue or cell sample with a hydrophilic or hydrophobic clearing agent, or by embedding the sample in a hydrogel and subjecting the embedded product to electrophoresis. As a clearing treatment method for the tissue or cell sample including using a hydrophilic clearing agent out of those methods, various methods have already been widely known to a person skilled in the art. In particular, the clearing treatment method herein is preferably a method known as a clear, unobstructed brain/body imaging cocktails and computational analysis (CUBIC) method (see Susaki et al., (2014) Cell; 157: 726-739; Tainaka K et al., (2014) Cell; 159: 911-924.).

A reagent to be used in the CUBIC method is commercially available, and the clearing treatment may be performed in accordance with a protocol provided by the manufacturer of the reagent, the protocol being recommended for each subject tissue ("Animal-clearing Reagent CUBIC", Tokyo Chemical Industry Co., Ltd., Tokyo, Japan). A typical method may be, for example, the following method. CUBIC-1 (reagent 1) is prepared as an aqueous solution of the mixture of 25 wt% of urea, 25 wt% of N,N,N',N'-tetrakis(2-hydroxypropyl)ethylenediamine, and 15 wt% of polyethylene glycol mono-p-isooctylphenyl ether/Triton X-100. CUBIC-2 (reagent 2) is prepared as an aqueous solution of the mixture of 50 wt% of sucrose, 25 wt% of urea, and 10 wt% of 2,2',2"-nitrilotriethanol. A procedure when the clearing treatment by the CUBIC method is performed in combination with the contact with the compound (I) is as described below. A fixed tissue sample is sufficiently washed with PBS, and is then immersed in a solution, which has been obtained by diluting the reagent 1 with distilled water to 1/2, at room temperature while being gently shaken. After that, the sample is immersed in the reagent 1 at room temperature for from 3 days to 7 days while being gently shaken. During the immersion, the reagent 1 is replaced with a new one at an interval of once per 1 to 2 days. After that, the sample is sufficiently washed with PBS, and is then immersed in PBS containing 20% to 30% (w/v) of sucrose. After that, a frozen block can be produced by using an embedding agent for producing a frozen tissue section (e.g., Sakura Finetek Japan Co., Ltd., TISSUE-TEK O.C.T. COMPOUND), and the sample can be stored for a long time period under the state in a freezer at -80°C. After the frozen block is thawed, the thawed product is sufficiently washed with PBS and is subjected to pretreatment as required, followed by whole-mount staining with the compound (I). After that, the stained product is treated with a linker as required, and is immersed in a solution obtained by diluting the reagent 2 with distilled water to 1/2, followed by gentle shaking at room temperature. After that, the resultant is immersed in the reagent 2 at room temperature for from 1 day to 3 days while being gently shaken. Three-dimensional imaging can be performed on the sample in such the state by using a fluorescence microscope.

With regard to the CUBIC reagent, in addition to CUBIC-1 and CUBIC-2 described above, various kinds of reagents including CUBIC-L, CUBIC-R+, CUBIC-B, CUBIC-HL, CUBIC-P, CUBIC-X1, and CUBIC-X2 exist, and are properly used in accordance with the kind of an organ. All of those reagents are included as CUBIC reagents applicable to the present invention.

In addition, the clearing treatment method applicable to the present invention is not limited to the CUBIC method. The other methods include: "BABB" (see Dobt HU et al., (2007) Nat Methods; 4: 331-336.), "3DISCO" (see Erturk A et al., (2012) Nat Protoc; 7: 1983-1995.), and "uDISCO" (see Pan C et al., (2016) Nat Methods; 13: 859-867.) each including using a hydrophobic clearing agent, and approaches serving as subtypes thereof; "Scale" (see Hama H et al., (2011) Nat Neurosci; 14: 1481-1488.) and "SeeDB" (see Ke MT et al., (2013) Nat Neurosci; 16: 1154-1161.) each including using a water-soluble clearing agent, and approaches serving as subtypes thereof; and "CLARITY" (see Chung K et al., (2013) Nature; 497: 332-7.) and "PACT" (see Tomer R et al., (2014) Nat Protoc; 9: 1682-97.) each of which is based on a principle in which a light-scattering body is removed by electrophoresis, and approaches serving as subtypes thereof.

### A-3. Contact

The compound (I) and the tissue or cell sample may be brought into contact with each other under any appropriate conditions in accordance with purposes. The compound (I) and the tissue or cell sample are typically brought into contact with each other by immersing the tissue or cell sample in a staining solution containing the compound (I). Contact conditions, such as the concentration of the compound (I) in the staining solution, an additive, a diluting liquid, a washing liquid, a contact time, and a contact temperature, may be appropriately selected by a person skilled in the art.

For example, when the compound (I) has a hydrazide group, a hydrazino group, or a hydroxylamino group, a condensation reaction between the substituent and an aldehyde is catalyzed by an aniline derivative or an indoline derivative, and the corresponding acylhydrazone, hydrazone, or oxime is efficiently formed (Organic Letters, 2020, 22, 6035-6040). Further, a reaction in which a benzimidazole structure is formed from a diaminobenzene structure and an aldehyde is accelerated by the use of a hydrogen peroxide solution (Synthesis, 2007, 4, 547-550), ammonium chloride (Indian Journal of Chemistry, 2013, 52B, 1152-1156), or sodium sulfite (Chemical Papers, 2018, 72, 1265-1276), or heating conditions (Molecular Diversity, 2015, 19, 263-272). A method for the reaction acceleration is not limited to the above-mentioned examples, and an additive or a staining condition may be appropriately selected for improving the efficiency of the reaction between the substituent X and the aldehyde.

After the contact with the compound (I), the tissue or cell sample may be washed by being immersed in a washing liquid such as PBS as required.

### A-4. Substance containing Glycol Group

A typical example of the "substance containing a glycol group" is a substance containing a sugar. Examples of the substance containing a sugar include glycogen, starch, mucopolysaccharides, glyceroglycolipids, and glycosphingolipids. An example of the glycol group is an α-glycol group.

### A-5. Applications

The fluorescent staining method according to the embodiment of the present invention enables specific staining of the substance containing a glycol group, and hence enables specific staining of a tissue, a lesion, or a structure containing the substance, preferably a tissue, a lesion, or a structure containing a large amount of the substance (as compared to any other tissue or cell). Examples of the tissue, the lesion, or the structure containing a large amount of the substance containing a glycol group include a muscle (e.g., a striated muscle tissue, a smooth muscle tissue, or a cardiac muscle tissue), mucus, a mucosal epithelium, a basement membrane (e.g., a renal glomerular basement membrane or a gastrointestinal tract mucosal basement membrane), an intracytoplasmic glycogen granule, a bacterium, a fungus, and a parasite. Examples of a disease for which the fluorescent staining method according to the embodiment of the present invention is useful include an inflammatory bowel disease, a cancer derived from a gland containing mucus, glomerulonephritis, cardiomyopathy, and an infectious disease. Accordingly, the term "fluorescent staining method for a substance containing a glycol group" as used herein may be appropriately rephrased as "fluorescent staining method for a tissue and/or a lesion containing a large amount of a substance containing a glycol group as compared to any other tissue or cell."

Herein, examples of the "inflammatory bowel disease" may include, but not limited to, ulcerative colitis and Crohn's disease.

Herein, examples of the "cancer" include, but not limited to, lung cancer, non-small cell lung cancer, small cell lung cancer, non-Hodgkin's lymphoma, adrenocortical carcinoma, AIDS-related cancer, AIDS-related lymphoma, childhood cerebellar astrocytoma, childhood cerebral astrocytoma, basal cell carcinoma, skin cancer (non-melanoma), biliary tract cancer, extrahepatic cholangiocarcinoma, intrahepatic cholangiocarcinoma, bladder cancer, bone and joint cancer, osteosarcoma and malignant fibrous histiocytoma, brain cancer, brain tumor, brainstem glioma, cerebellar astrocytoma, glioma, cerebral astrocytoma/malignant glioma, ependymoma, medulloblastoma, supratentorial primitive neuroectodermal tumor, visual pathway and hypothalamic glioma, head and neck cancer, metastatic squamous neck cancer, bronchial adenoma/carcinoid, carcinoid tumor, nervous system lymphoma, central nervous system cancer, central nervous system lymphoma, neuroblastoma, childhood cancer, Sezary syndrome, extracranial germ cell tumor, extragonadal germ cell tumor, extrahepatic cholangiocarcinoma, eye cancer, intraocular melanoma, retinoblastoma, salivary gland cancer, oral cancer, oral cavity cancer, oropharyngeal cancer, tongue cancer, esophageal cancer, gastric cancer, gastrointestinal carcinoid tumor, gastrointestinal stromal tumor (GIST), small intestine cancer, colon cancer, colorectal cancer, rectal cancer, anorectal cancer, anus cancer, germ cell tumor, hepatocellular (liver) cancer, Hodgkin's lymphoma, laryngeal cancer, pharyngeal cancer, hypopharyngeal cancer, nasopharyngeal cancer, nasal sinus and nasal cavity cancer, throat cancer, pancreatic islet cell tumor (endocrine pancreas), pancreatic cancer, parathyroid cancer, liver cancer, gallbladder cancer, appendix cancer, renal cancer, urethral cancer, transitional cell carcinoma of the renal pelvis and ureter and other urinary organ cancers, squamous cell carcinoma, penile cancer, testicular cancer, thymoma, thymoma and thymic carcinoma, thyroid cancer, prostate cancer, ovarian cancer, ovarian epithelial cancer, ovarian low malignant potential tumor, ovarian germ cell tumor, gestational trophoblastic tumor, breast cancer, uterine cancer, uterine sarcoma, cervical cancer, endometrial cancer, uterine corpus cancer, vaginal cancer, vulvar cancer, Wilms tumor, skin cancer (non-melanoma), skin cancer (melanoma), pheochromocytoma, Merkel cell skin cancer, mesothelioma, malignant mesothelioma, multiple endocrine neoplasia syndrome, myelodysplastic syndrome, myelodysplastic/myeloproliferative diseases, pineoblastoma and pituitary tumor, plasmacytoma, pleuropulmonary blastoma, retinoblastoma, rhabdomyosarcoma, sarcoma, Ewing's tumor, soft tissue sarcoma, soft tissue sarcoma, mycosis fungoides, and Kaposi's sarcoma. Examples of the cancer derived from a gland containing mucus out of those cancers include non-small cell lung cancer (mucinous lung adenocarcinoma), skin cancer (extramammary Paget's disease), gastric cancer, small intestine cancer, colon cancer, colorectal cancer, rectal cancer, breast cancer (Paget's disease of the breast), ovarian cancer, pancreatic cancer, liver cancer, gallbladder cancer, and appendix cancer.

Herein, examples of the "glomerulonephritis" may include, but not limited to, a minimal change disease, membranous nephropathy, mesangial proliferative nephritis, endocapillary proliferative glomerulonephritis, membranoproliferative glomerulonephritis, extracapillary proliferative glomerulonephritis, sclerosing glomerulonephritis, lupus nephritis, IgA nephropathy, purpura nephritis (Henoch-Schonlein purpura), and anti-GBM glomerulonephritis (Goodpasture syndrome).

Herein, examples of the "cardiomyopathy" may include, but not limited to, dilated cardiomyopathy, hypertrophic cardiomyopathy, restrictive cardiomyopathy, and arrhythmogenic cardiomyopathy.

Herein, examples of the "infectious disease" to be assumed include, but not limited to, diseases caused by infection with the following bacteria, fungi, and parasites. Specific examples thereof include Streptococcus (e.g., group A β-hemolytic streptococcus or pneumococcus), *Staphylococcus aureus* (MSSA or MRSA), *Staphylococcus epidermidis,* Enterococcus, Listeria, *Neisseria meningitidis, Neisseria gonorrhoeae,* pathogenic *Escherichia coli* (e.g., O157:H7), Klebsiella (*Klebsiella pneumoniae*), Proteus, *Bordetella pertussis, Pseudomonas aeruginosa,* Serratia, Citrobacter, Acinetobacter, Enterobacter, Mycoplasma, Clostridium, Tuberculosis/non-tuberculous mycobacteria, Cholera, Plague, Diphtheria, Shigella, Scarlet fever, Anthrax, syphilis, tetanus, leprosy, legionella pneumonia (legionnaires' disease), leptospirosis, Lyme disease, tularemia, Q fever, rickettsia, chlamydia, aspergillosis, candidiasis, cryptococcosis, trichophytosis, histoplasmosis, pneumocystis pneumonia, amoebic dysentery, malaria, toxoplasmosis, leishmaniasis, cryptosporidium, echinococcosis, Schistosoma japonicum, filariasis, ascariasis, and fish tapeworm infection.

### B. Detection Method

According to another aspect of the present invention, there is provided a method of detecting a substance containing a glycol group in a tissue or cell sample, the method including: subjecting the substance containing a glycol group in the tissue or cell sample to fluorescent staining by the method described in the section A; and measuring fluorescence derived from the compound (I).

The fluorescence derived from the compound (I) may be measured, for example, as follows: the excitation light of the compound (I) is applied to the tissue or cell sample subjected to fluorescent staining, followed by the measurement of the intensity of fluorescence emitted by the compound (I) with a fluorescence microscope. It can be recognized that the substance containing a glycol group is present in a site where fluorescence, which has an intensity stronger than a fluorescence intensity measured when no excitation light is applied, is measured, and the abundance of the substance can be detected in accordance with the range and/or intensity of the fluorescence.

The tissue or cell sample, the substance containing a glycol group, and a method of fluorescently staining the substance are as described in the section A.

### C. Visualization Method

According to another aspect of the present invention, there is provided a method of visualizing a tissue or a cell containing a substance containing a glycol group, the method including: subjecting the substance containing a glycol group in a tissue or cell sample to fluorescent staining by the method described in the section A; and measuring and imaging fluorescence derived from the compound (I).

The fluorescence derived from the compound (I) may be measured and imaged, for example, as follows: the excitation light of the compound (I) is applied to the tissue or cell sample subjected to fluorescent staining, followed by the measurement of the intensity of fluorescence emitted by the compound (I) with a fluorescence microscope; and the resultant fluorescence intensity data is imaged with image analysis software. Any one of a two-dimensional image or a three-dimensional image may be produced in accordance with purposes. Known software, such as Imaris, ImageJ, or cellSens, may be used as the image analysis software.

The excitation light of the compound (I) has a wavelength in the range of from about 400 nm to about 520 nm, and is hence excellent in permeability into the tissue or cell sample having a thickness. Accordingly, the visualization method according to the embodiment of the present invention enables suitable visualization of a tissue or a cell, which contains a substance containing a glycol group and has a three-dimensional stereoscopic structure. The tissue or the cell is preferably subjected to clearing treatment.

The tissue or the cell to which the visualization method according to the embodiment of the present invention is preferably applied is, for example, a mucosal epithelium, a basement membrane, or a fungus. For example, numberless tubular intruding portions (crypts) are present in a mucosal epithelium of a mammal (e.g., a small intestinal or colonic mucosal epithelium), and each of the crypts includes several kinds of cells including a goblet cell that produces mucus. The visualization method according to the embodiment of the present invention achieves specific fluorescent staining of the goblet cell containing the mucus, and as a result, enables clear visualization of the shapes of the individual crypts.

The tissue or the cell containing the substance containing a glycol group, and a method of fluorescently staining the substance are as described in the section A.

### D. Determination Method

According to another aspect of the present invention, there is provided a method of determining a tissue and/or a lesion containing a large amount of a substance containing a glycol group in a tissue or cell sample, the method including: subjecting the substance containing a glycol group in the tissue or cell sample to fluorescent staining by the method described in the section A; and determining that the tissue and/or the lesion is present in a stained site. The present invention also provides a method of providing information for determining a tissue and/or a lesion containing a large amount of a substance containing a glycol group in a tissue or cell sample, the method including: subjecting the substance containing a glycol group in the tissue or cell sample to fluorescent staining by the method described in the section A; and providing the information for determining that the tissue or the lesion is present in a stained site. The tissue and the lesion each containing a large amount of the substance containing a glycol group are as described in the section A.

The determination of the tissue and/or the lesion containing a large amount of the substance containing a glycol group may be any determination as long as the determination is determination, which can be performed from two-dimensional or three-dimensional image data on the presence of the substance containing a glycol group, such as the determination of the amount, symptom degree, position, and/or shape of the tissue or the lesion in addition to determination as to whether or not the tissue or the lesion is present.

Although the tissue and/or the lesion containing a large amount of the substance containing a glycol group can be simply determined from the presence or absence of staining in some cases, the determination may be performed in comprehensive consideration of, for example, the position at which the substance containing a glycol group is detected and its amount (the position at which fluorescence is detected and its intensity), the site from which the tissue or cell sample is derived, information on a patient, and general findings in the field of pathology concerning the pattern of presence of the substance containing a glycol group as required.

For example, when the tissue or cell sample is a cancer-excised tissue fragment of a cancer patient, and a cancer is targeted as the lesion, the presence of the cancer in the tissue or cell sample can be determined by examining the position at which the substance containing a glycol group is present and its amount, the position and the amount representing the accumulation of the substance containing a glycol group, the accumulation being characteristic of the cancer.

In addition, in, for example, an inflammatory bowel disease, a lesion characteristic of the disease includes crypt distortion. According to analysis including using the approach of the present invention, through a three-dimensional observation of the crypt distortion, distortion of such a shape that a plurality of crypts are involved has been found as a lesion specific to the inflammatory bowel disease, in particular, a lesion specific to ulcerative colitis. Accordingly, when the tissue or cell sample is a colonic mucosal tissue derived from an individual that may suffer from the inflammatory bowel disease, the presence of the lesion can be determined by analyzing the shapes of the crypts.

Specifically, the presence of the crypt distortion serving as a lesion characteristic of the inflammatory bowel disease can be determined by: subjecting the colonic mucosal tissue to the fluorescent staining method described in the section A to provide a three-dimensional image of crypts through the staining of a goblet cell containing mucus and a basement membrane in a mucosal epithelium; and three-dimensionally analyzing the shapes of the crypts on the basis of the resultant three-dimensional image.

The three-dimensional analysis of the shape of each of the crypts may be performed by, for example, determining the volume, ellipticity, or tortuosity of the crypt from the three-dimensional image data thereof with image analysis software. In ulcerative colitis, the volume of the crypt tends to show a value increased as compared to those in non-specific colitis and Crohn's disease. In each of ulcerative colitis and Crohn's disease, the ellipticity tends to show a value reduced as compared to that in non-specific colitis. The tortuosity tends to show the following value: its value is highest in ulcerative colitis, and reduces in the order of Crohn's disease and non-specific colitis. Accordingly, in, for example, the case where the ellipticity is lower than a predetermined criterion (e.g., the criterion range of an ellipticity in an individual suffering from non-specific colitis), or the case where the tortuosity is higher than a predetermined criterion (e.g., the criterion range of a tortuosity in the individual suffering from non-specific colitis), it can be determined that the crypt distortion is present as a lesion characteristic of the inflammatory bowel disease.

In addition, the possibility that the individual from which the sample is derived suffers from the inflammatory bowel disease can be evaluated by quantifying or comprehensively evaluating the crypt distortion. As a specific example, the following determination (diagnosis) can be made by using the volume, ellipticity, and tortuosity of the crypt as parameters: when a crypt having a volume exceeding a predetermined criterion (e.g., the criterion range of a volume in non-specific colitis or Crohn's disease), an ellipticity lower than a predetermined criterion (e.g., a criterion range in an individual suffering from non-specific colitis), and a tortuosity higher than a predetermined criterion (e.g., the criterion range of a tortuosity in Crohn's disease) is observed, the individual from which the sample is derived is likely (or highly likely) to suffer from ulcerative colitis. As another specific example, the following determination (diagnosis) can be made: when a point in accordance with a degree (e.g., a higher point along with a larger volume, a higher point along with a lower ellipticity, or a higher point along with a higher tortuosity) is set for each of the volume, the ellipticity, and the tortuosity, and a predetermined number or more, or a predetermined ratio or more, of crypts in each of which the total of the points is more than a predetermined criterion are observed, the individual from which the sample is derived is likely (or highly likely) to suffer from ulcerative colitis.

That is, according to another aspect of the present invention, there is provided a method of determining a possibility that an individual suffers from an inflammatory bowel disease, such as ulcerative colitis or Crohn's disease, the method including: subjecting a substance containing a glycol group in a colonic mucosal tissue sample derived from the individual to fluorescent staining by the method described in the section A; measuring and imaging fluorescence derived from the compound (I) to provide a three-dimensional image of a crypt; and three-dimensionally analyzing the shape of the crypt on the basis of the resultant three-dimensional image. As described above, the three-dimensional analysis of the shape of the crypt may be performed by quantifying or comprehensively evaluating crypt distortion through use of the volume, ellipticity, and tortuosity of the crypt as parameters.

### E. Screening Method

According to another aspect of the present invention, there is provided a method of screening a therapeutic agent for a disease involving a lesion of a tissue or a cell containing a substance containing a glycol group, the method including: administering a therapeutic agent candidate to an individual suffering from the disease; collecting a tissue or cell sample in which the lesion occurs from the individual; subjecting the substance containing a glycol group in the tissue or cell sample to fluorescent staining by the method described in the section A; measuring and imaging fluorescence derived from the compound (I) to visualize the tissue or the cell containing the substance containing a glycol group; and determining a therapeutic effect of the therapeutic agent candidate on the basis of an image of the tissue or the cell containing the substance containing a glycol group that has been visualized.

Examples of the disease involving the lesion of the tissue or the cell containing the substance containing a glycol group include an inflammatory bowel disease, a cancer derived from a gland containing mucus, glomerulonephritis, cardiomyopathy, and an infectious disease. In addition, examples of the tissue or the cell in which the lesion occurs include a muscle (e.g., a striated muscle tissue, a smooth muscle tissue, or a myocardial tissue), mucus, a mucosal epithelium, a basement membrane (e.g., a renal glomerular basement membrane or a gastrointestinal mucosal basement membrane), an intracytoplasmic glycogen granule, a bacterium, a fungi, and a parasite.

The individual suffering from the disease described above is a human or a mammal except the human such as an experimental animal, and examples thereof include a mouse, a rat, a guinea pig, a gerbil, a hamster, a ferret, a rabbit, a dog, a miniature pig, and a monkey. The dose and administration method of the therapeutic agent candidate may be appropriately set in accordance with, for example, the therapeutic agent and the condition of the individual.

A method of fluorescently staining the substance containing a glycol group in the tissue or cell sample, and a method of measuring and imaging the fluorescence derived from the compound (I) to visualize the tissue or the cell containing the substance containing a glycol group are as described in the section A and the section C, respectively.

The therapeutic effect of the therapeutic agent candidate is determined on the basis of the image of the tissue or the cell containing the substance containing a glycol group that has been visualized. In, for example, the case where in the visualized image, a lesion that occurs along with a disease to be treated (the lesion of the tissue or the cell containing the substance containing a glycol group) is alleviated or reduced as compared to that before the administration of the therapeutic agent candidate, or the case where in the image, the progress of the disease (lesion) is suppressed as compared thereto, it can be determined that the therapeutic agent candidate is likely (or highly likely) to have a therapeutic effect on the disease. In addition, in, for example, the case where the lesion that occurs along with the disease to be treated is not changed or is increased as compared to that before the administration of the therapeutic agent candidate, it can be determined that the therapeutic agent candidate is likely (or highly likely) to have no therapeutic effect on the disease.

### F. Composition for Fluorescent Staining

According to another aspect of the present invention, there is provided a composition for fluorescent staining including a compound (I) or a salt thereof, wherein the composition is used for visualizing a tissue or a cell containing a substance containing a glycol group. As described above, the compound (I) can specifically stain the substance containing a glycol group, and is excellent in permeability into the tissue or the cell. Accordingly, when the tissue or the cell, which contains the substance containing a glycol group and is preferably subjected to clearing treatment, and the compound (I) are brought into contact with each other, the substance containing a glycol group is stained inside the tissue or the cell, and as a result, the tissue or the cell containing the substance can be visualized.

The object to be visualized may be any appropriate tissue or cell containing a substance containing a glycol group, and is preferably a tissue or a cell having a three-dimensional stereoscopic structure, more preferably, for example, a muscle (e.g., a striated muscle tissue, a smooth muscle tissue, or a myocardial tissue), a mucosal epithelium, a basement membrane (e.g., a renal glomerular basement membrane or a gastrointestinal mucosal basement membrane), an intracytoplasmic glycogen granule, a bacterium, a fungus, or a parasite, still more preferably a colonic mucosal epithelium (intestinal crypt) or a fungus.

The composition for fluorescent staining may further include any appropriate constituent component, such as a clearing agent, a diluent, or a turbid liquid agent, as required. The clearing agent is preferably a hydrophilic clearing agent.

The composition for fluorescent staining may be in the form of a mixed liquid in which its respective constituent components are mixed, or may be in a form (kit) in which the constituent components are each, or part of the constituent components are, individually packaged. According to the latter form, the respective constituent components may be mixed by a user.

### G. Diagnostic Composition

According to another aspect of the present invention, there is provided a diagnostic composition for a disease involving a lesion of a tissue or a cell containing a substance containing a glycol group, including a compound (I) or a salt thereof. As described above, the compound (I) can specifically stain the substance containing a glycol group, and is excellent in permeability into the tissue or the cell. Accordingly, when the tissue or the cell, which contains the substance containing a glycol group and is preferably subjected to clearing treatment, is brought into contact with the compound (I), the substance containing a glycol group is stained in the tissue or the cell, and hence the tissue or the cell is visualized. When such a determination method as described in the above-mentioned section D is applied to an image of the visualized tissue or cell, the presence or absence, number, incidence, and the like of lesions can be determined, and the possibility that an individual suffers from the disease involving the lesion can be judged on the basis of the determination. That is, according to the diagnostic composition according to the embodiment of the present invention, information for diagnosing the disease involving the lesion of the tissue or the cell containing the substance containing a glycol group can be provided through the visualization of the tissue or the cell.

The judgment (i.e., diagnosis) of the possibility that the individual suffers from the above-mentioned disease may be performed with artificial intelligence (AI) as well as by a doctor. The diagnosis with the AI may be performed, for example, by using an image diagnosis-assisting program that analyzes the image of the visualized tissue or cell in accordance with predetermined criteria and calculates the possibility that the individual suffers from the above-mentioned disease on the basis of the result of the analysis. Alternatively, the possibility that the individual suffers from the above-mentioned disease may be judged as follows: the image of the visualized tissue or cell is analyzed with the AI in accordance with predetermined criteria; and the doctor judges the possibility on the basis of the result of the analysis.

The disease to be diagnosed only needs to be a disease involving the lesion of the tissue or the cell containing the substance containing a glycol group. Specific examples thereof may similarly include the diseases described in the section E. Of those, an inflammatory bowel disease is preferred, and ulcerative colitis or Crohn's disease is more preferred. For example, crypt distortion and/or the degree of the crypt distortion may be used as an analysis criterion for the diagnosis of the inflammatory bowel disease.

The diagnostic composition may further include any appropriate constituent component, such as a clearing agent, a diluent, or a turbid liquid agent, as required. The clearing agent is preferably a hydrophilic clearing agent.

The diagnostic composition may be in the form of a mixed liquid in which its respective constituent components are mixed, or may be in a form (kit) in which the constituent components are each, or part of the constituent components are, individually packaged. According to the latter form, the respective constituent components may be mixed by a user.

### Examples

The present invention is described in more detail below by way of Examples. However, Examples do not limit the scope of the present invention. The entirety of literatures cited throughout the description of the present application is incorporated herein by reference.

### [Example 1] Synthesis of Compound 1

Compound 1 was synthesized through steps described in the following scheme.

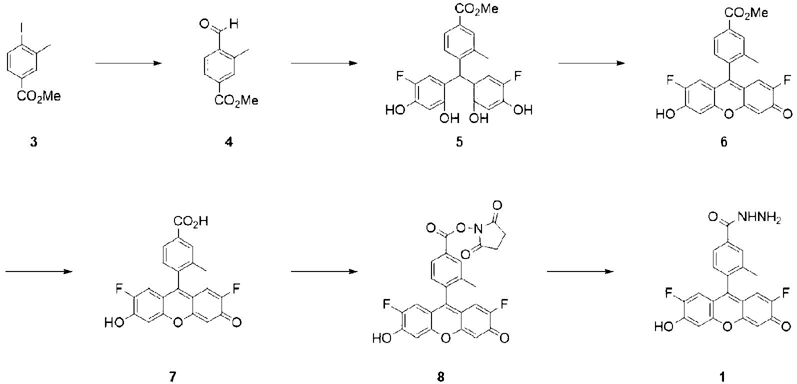

2 M iPrMgCl/THF (2.6 mL, 5.27 mmol) was dropped into a solution of Compound 3 (364 mg, 1.32 mmol) in THF (13 mL) at -15°C. After the mixture had been stirred for 1.5 hours, DMF (0.5 ml, 6.59 mmol) was added to the mixture, and the whole was stirred at room temperature for 2 hours. 1 M HCl was added to the mixture, and the whole was stirred for 1 hour. After that, the reaction solution was extracted with a mixed solution containing hexane and ethyl acetate at 3:1. The organic phase was washed with a saturated saline solution, and was dried with Na₂SO₄. The solvent was evaporated under reduced pressure. Thus, Compound 4 that was a crude product was obtained as a colorless oil. The compound was immediately used in the next reaction without being further purified.

Methanesulfonic acid (1.3 ml) was added to a solution of Compound 4 obtained in the foregoing and 4-fluororesorcinol (320 mg, 2.50 mmol) in a mixed solvent (13 ml) containing CH₂Cl₂ and Et₂O at 1:1 at room temperature, and the mixture was stirred for 18.5 hours. Saturated sodium hydrogen carbonate was carefully added to neutralize the mixture, and then the pH of the neutralized product was adjusted to about 3 with concentrated hydrochloric acid. The resultant was extracted with ethyl acetate, and the organic phase was washed with a saturated saline solution and dried with Na₂SO₄. The solvent was evaporated under reduced pressure, and the resultant residue was purified by silica gel column chromatography (CH₂Cl₂/MeOH=20_{→}15) to provide Compound 5 (382 mg, 70% (2 steps)).
¹H NMR (400 MHz, DMSO-d6) δ 9.56 (2H, s), 9.17 (2H, s), 7.72 (1H, d, J=2.0 Hz), 7.68 (1H, dd, J=8.0, 2.0 Hz), 6.85 (1H, d, J=8.0 Hz), 6.45 (2H, d, J=8.0 Hz), 6.19 (2H, d, J=12.0 Hz), 5.83 (1H, s), 3.81 (3H, s), 2.15 (3H, s)

PTS·H₂O (25.1 g, 132 mmol) was added to a solution of Compound 5 (5.5 g, 13.2 mmol) in toluene (264 ml), and the mixture was stirred at 120°C for 24 hours. The reaction solution was cooled to room temperature, and a 2 M aqueous solution of potassium hydroxide (100 mL) was added to the solution, followed by stirring for 5 hours. The reaction solution was washed with diethyl ether, and its pH was adjusted to about 3 with concentrated hydrochloric acid. The resultant was extracted with ethyl acetate, and the organic phase was dried with Na₂SO₄. The solvent was evaporated under reduced pressure, and the resultant residue was purified by reverse-phase column chromatography (H₂O (1% Et₃N)/CH₃CN=90:10), followed by the removal of triethylamine with hydrochloric acid. Thus, Compound 7 was obtained (412 mg, 8.2% (2 steps)).
¹H NMR (400 MHz, DMSO-d6) δ 8.05 (1H, s), 7.97 (1H, d, J=8.0 Hz), 7.41 (1H, d, J=8.0 Hz), 6.78 (2H, br-s), 6.60 (2H, d, J=11.2 Hz), 2.09 (3H, s)

A solution of DCC (23.9 mg, 116 mmol) in THF (2 ml) was dropped into a solution of Compound 7 (34.1 mg, 89.1 mmol) and N-hydroxysuccinimide (15.3 mg, 134 mmol) in THF (7 ml). The reaction solution was stirred at room temperature for 5 hours, and the solvent was evaporated under reduced pressure. The resultant residue was purified by silica gel column chromatography (CH₂Cl₂/MeOH=50_{→}10) to provide Compound 8 as an orange solid (37.0 mg, 86%).
¹H NMR (400 MHz, CD₃OD) δ 8.29 (1H, s), 8.22 (1H, d, J=8.8 Hz), 7.54-7.49 (1H, m), 6.91 (2H, d, J=6.8 Hz), 6.80-6.74 (2H, m), 2.95 (4H, s), 2.19 (3H, s)

Hydrazine monohydrate (0.0136 mL, 0.280 mmol) was added to a solution of Compound 8 (13.4 mg, 28.0 mmol) in DMF (0.56 ml), and the mixture was stirred at room temperature for 2 hours. CH₂Cl₂ (5 mL) was added to the reaction solution, and the precipitated solid was filtered out. The precipitated solid was dissolved in 2 M hydrochloric acid, and the solution was purified with a reverse-phase silica gel column to provide Compound 1 (12.0 mg, 99%).
¹H NMR (400 MHz, CDCl₃) δ 8.02 (1H, s), 7.93 (1H, dd, J=8.0, 1.6 Hz), 7.48 (1H, d, J=8.0 Hz), 6.87 (2H, br-s), 6.63 (2H, d, J=10.8 Hz), 2.11 (3H, s)
HRMS (ESI⁺): calcd for [M+H]⁺, 397.0994; found, 397.0973 (-2.1 mmu)

### [Example 2] Synthesis of Compound 2

Compound 2 was synthesized through steps described in the following scheme.

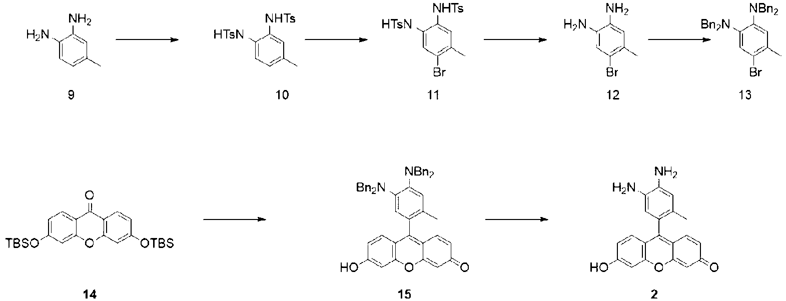

p-Toluenesulfonyl chloride (30.0 g, 157 mmol) was added to a solution of Compound 9 (8.01 g, 65.5 mmol) and pyridine (20 ml, 250 mmol) in dichloromethane (150 mL) at 0°C, and the mixture was stirred at room temperature for 16 hours. The reaction solution was washed with a 2 M aqueous solution of hydrochloric acid and distilled water, and was evaporated under reduced pressure. The resultant residue was recrystallized with ethanol to provide Compound 10 as a white crystal (25.9 g, 920). ¹H NMR (400 MHz, CDCl₃) δ 7.60 (2H, dd, J=8.8 Hz), 7.53 (2H, d, J=8.8 Hz), 7.23 (2H, d, J=3.6 Hz), 7.21 (2H, d, J=3.6 Hz), 7.03 (1H, s), 6.93 (1H, br-s), 6.79 (1H, dd, J=8.4, 2.0 Hz), 6.67 (1H, d, J=8.0 Hz), 6.44 (1H, br-s), 2.39 (6H, s), 2.19 (3H, s)

Bromine (4.0 mL, 78.2 mmol) was added to a solution of Compound 10 (25.9 g, 60.2 mmol) and sodium acetate (8.89 g, 108 mmol) in acetic acid (200 mL) at 0°C, and the mixture was heated to reflux for 3 hours. After the temperature of the reaction solution had been returned to room temperature, distilled water was added to the solution, and the produced precipitate was filtered. The resultant crude product was recrystallized with ethanol to provide Compound 11 as a white crystal (20.4 g, 67%).
¹H NMR (400 MHz, CDCl₃) δ 7.58 (2H, dd, J=8.4, 1.6 Hz), 7.55 (2H, dd, J=8.4, 1.6 Hz), 7.29-7.20 (4H, m), 7.00 (1H, d, J=1.6 Hz), 6.93 (1H, br-s), 6.86 (1H, br-s), 6.50 (1H, br-s), 2.40 (6H, s), 2.20 (3H, d, J=1.6 Hz)

Concentrated sulfuric acid (10 mL) was added to Compound 11 (10.2 g, 7.91 mmol), and the mixture was stirred under heating at 100°C for 30 minutes. The reaction solution was cooled in an ice bath, and its pH was adjusted to 10 with a 2 M aqueous solution of sodium hydroxide, followed by extraction with dichloromethane. The organic phase was dried with sodium sulfate, and then the solvent was evaporated under reduced pressure. Thus, Compound 12 was obtained as a pale yellow solid (2.22 g, 55%).
¹H NMR (400 MHz, DMSO-d6) δ 6.66 (1H, s), 6.43 (1H, s), 4.50 (4H, s), 2.08 (3H, s)

Sodium carbonate (968 mg, 9.13 mmol) and benzyl bromide (0.54 mL, 4.57 mmol) were added to a solution of Compound 12 (153 mg, 0.761 mmol) in DMF (3 mL), and the mixture was stirred at 60°C for 16 hours. After the temperature of the reaction solution had been returned to room temperature, distilled water was added to the solution, and the mixture was extracted with ethyl acetate. The organic phase was dried with sodium sulfate, and then the solvent was evaporated under reduced pressure. The resultant residue was purified by silica gel column chromatography (developing solvent: hexane/ethyl acetate=15/1) to provide Compound 13 as a white solid (345 mg, 81%) .
¹H NMR (400 MHz, CDCl₃) δ 7.29-7.19 (12H, m), 7.16-7.07 (8H, m), 6.94 (1H, s), 6.63 (1H, s), 4.37 (4H, s), 4.35 (4H, s), 2.23 (3H, s)
HRMS (ESI⁺): calcd for [M+H]⁺, 561.1900; found, 561.1836 (-6.4 mmu)

Compound 14 was synthesized by a method described in a literature (J. AM. Chem. Soc. (2005) 127: 4888).

sec-BuLi (0.48 mL, 0.501 mmol) was added to a solution of Compound 13 (219 mg, 0.501 mmol) in THF (4 mL) under a nitrogen atmosphere at -78°C, and the mixture was stirred. Compound 14 (209 mg, 0.458 mmol) was added to the reaction solution, and the temperature of the mixture was increased to room temperature, followed by stirring for 20 minutes. A 2 M aqueous solution of hydrochloric acid was added to the reaction solution, and the mixture was stirred at room temperature for 30 minutes, followed by extraction with dichloromethane. The organic phase was dried with sodium sulfate, and then the solvent was evaporated under reduced pressure. The residue was purified by silica gel column chromatography (developing solvent: dichloromethane/methanol=98/2) to provide Compound 15 as an orange solid (140 mg, 44%).
¹H NMR (400 MHz, CDCl₃) δ 7.32-7.23 (6H, m), 7.21-7.10 (10H, m), 7.07-7.01 (4H, m), 6.87 (2H, s), 6.86 (2H, d, J=9.2 Hz), 6.79 (2H, dd, J=9.2, 2.4 Hz), 6.76 (1H, s), 6.43 (1H, s), 4.52 (4H, s), 4.33 (4H, s), 1.82 (3H, s)
HRMS (ESI⁺): calcd for [M+H]⁺, 693.3112; found, 693.3099 (-1.3 mmu)

Pd/C (10 mg) was added to a solution of Compound 15 (72.9 mg, 0.105 mmol) in a mixed solvent (3 mL) containing MeOH and THF at 1:1, and the mixture was stirred under a hydrogen atmosphere at room temperature for 2 days. The reaction solution was subjected to Celite filtration, and the filtrate was evaporated under reduced pressure. The residue was dissolved in a mixed solution (1 mL) containing dichloromethane and MeOH at 4:1, and chloranil (51.6 mg, 0.210 mmol) was added to the solution, followed by stirring at room temperature for 30 minutes. After that, the reaction solution was evaporated under reduced pressure. The residue was purified by silica gel column chromatography (developing solvent: dichloromethane/methanol=98/2) to provide Compound 2 as an orange solid (5.3 mg, 12%).
¹H NMR (400 MHz, CD₃OD) δ 7.17 (2H, d, J=8.8 Hz), 6.71 (1H, s), 6.50-6.60 (4H, m), 6.52 (1H, s), 1.83 (3H, s)
HRMS (ESI⁺): calcd for [M+H]⁺, 333.1234; found, 333.1223 (-1.1 mmu)

### [Example 3] Fluorescence Emission by DAF-2

When propionaldehyde was caused to act on DAF-2 (product model number: SK1001-01, Goryo Chemical, Inc.), Compound 16 below was obtained. It was recognized that Compound 16 was fluorescent and its fluorescence intensity increased along with the formation of a benzimidazole ring. Although the fluorescence of DAF-2 before the reaction with the aldehyde was quenched by its diaminobenzene structure, Compound 16 that was a product of the reaction between DAF-2 and the aldehyde showed a fluorescence property (Table 1). The formation of a covalent bond with the aldehyde enables detection by fluorescence emission.

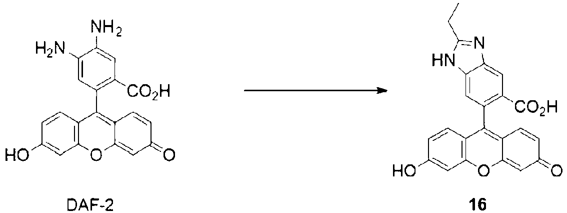

**Table 1**

| | Molar extinction coefficient | Quantum yield |
|---|---|---|
| DAF-2¹⁾ | 79,000 (λabs: 486 nm) | 0.005 |
| Compound 16²⁾ | 63,000 (λabs: 489 nm) | 0.85 |

1) Data in 100 mM sodium phosphate buffer, pH 7.4, cited from Analytical Chemistry, 1998, 70, 2446
2) Data in 10 mM sodium phosphate buffer, pH 7.4

### [Example 4] Changes in Light Characteristics of DAF-2 and Compound 2

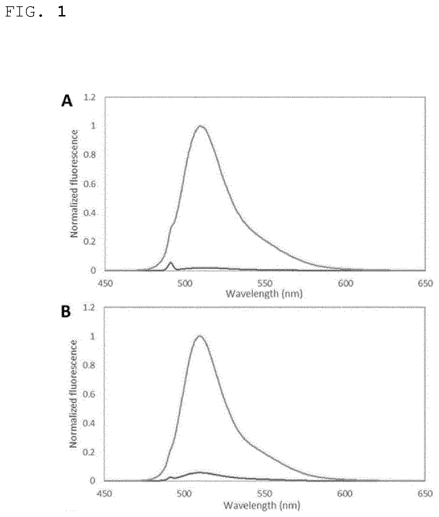

Propionaldehyde was added to a 10 mmol/L sodium phosphate buffer (pH: 7.4) containing 0.02 mmol/L of each of DAF-2 and Compound 2 so that its final concentration became 0.10 mmol/L, followed by stirring at room temperature for 4 days. The absorption and fluorescence spectra of each of the solutions before the addition of the aldehyde and after the addition were measured. As a result, as shown in each of FIGS. **1****,** a fluorescence intensity after the addition of the aldehyde (the spectrum having a large peak) showed an increase as compared to that before the addition (the spectrum having a small peak).

### [Example 5] Staining with DAF-2 and Compound 2

A fluorescent probe containing a diaminobenzene structure, such as DAF-2 or Compound 2, was examined for its staining property in the mucus of a goblet cell by using a frozen sliced section of a human colonic mucosal tissue as a positive control analyte. Specifically, a sliced frozen section was cut out of the colonic mucosal tissue after its formalin fixation, and was stained with a group of candidate fluorescent probes and washed with phosphate buffered saline. After that, an image of the section was taken with a confocal fluorescence microscope.

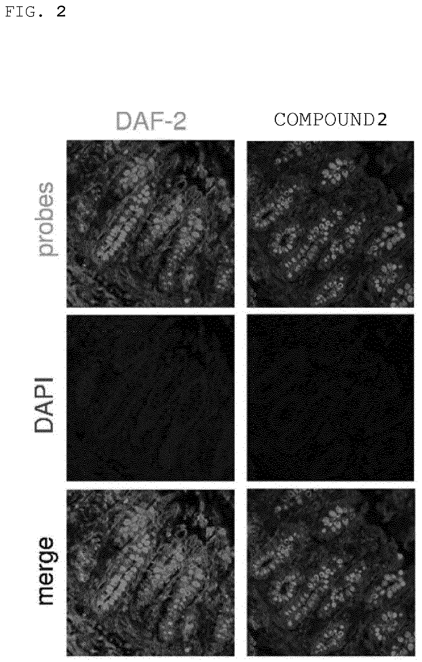

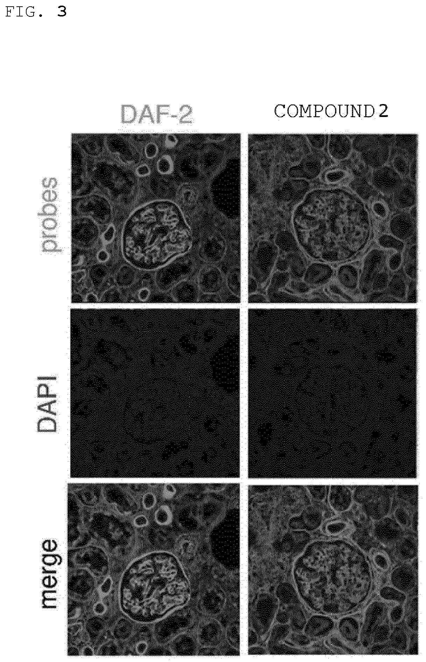

The mucus of the goblet cell of the colonic mucosa was able to be clearly stained through staining with DAF-2 and Compound 2 (FIG. **2**). Specifically, the human colonic mucosal tissue sample after the formalin fixation was sufficiently washed with PBS, and was then immersed in PBS containing 30% (w/v) of sucrose, followed by the production of a frozen block with TISSUE-TEK O.C.T. COMPOUND. The frozen block was sliced into a thickness of 8 um to produce a frozen section. The frozen section was washed with PBS, and was then stained with a solution, which had been obtained by diluting DAF-2 (50 µM) or Compound 2 (50 µM) with PBS, at room temperature for 1 day. After that, the stained product was sufficiently washed with PBS. Then, the washed product was mounted with a water-soluble mounting medium, and an image thereof was taken with a confocal microscope. Similarly, the basement membrane of a renal glomerulus was able to be clearly visualized through staining with DAF-2 and Compound 2 (FIG. **3**).

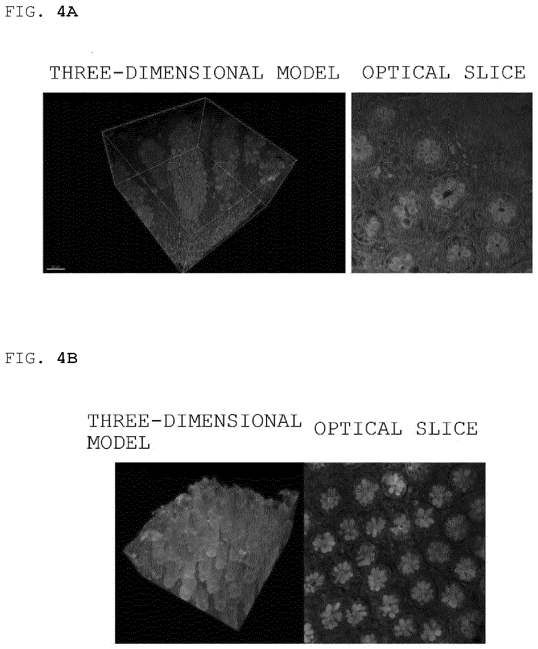

In addition, combined use of the staining with DAF-2 or Compound 2 and a tissue clearing method based on a CUBIC reagent enabled the 3D imaging of a colonic mucosal epithelium. Specifically, the human colonic mucosal tissue sample after the formalin fixation was sufficiently washed with PBS, and was then immersed in a solution, which had been obtained by diluting a CUBIC-L reagent with distilled water to 1/2, at 37°C while being gently shaken. After that, the sample was immersed in the CUBIC-L reagent, and was treated at 37°C while being gently shaken. During the treatment, the CUBIC-L reagent was replaced with a new one at an interval of once per 1 to 2 days. After that, the sample was sufficiently washed with PBS, and was then immersed in PBS containing 30% (w/v) of sucrose. After that, a frozen block of the sample was produced by using TISSUE-TEK O.C.T. COMPOUND. The frozen block was thawed, and was then sufficiently washed with PBS and subjected to pretreatment with a 1% aqueous solution of periodic acid, followed by staining with a solution, which had been obtained by diluting DAF-2 (50 µM) with PBS, at room temperature for 3 days. After that, the stained product was sufficiently washed with PBS, and was then immersed in a solution obtained by diluting a CUBIC-R+ reagent with distilled water to 1/2, followed by gentle shaking at room temperature. After that, the resultant was immersed in the CUBIC-R+ reagent at room temperature for 1 day while being gently shaken. An image of the resultant was taken with a confocal microscope. A three-dimensional model obtained from the resultant data with Imaris manufactured by Bitplane Inc. is shown in FIG. **4A****.** A confocal micrograph when the staining with Compound 2 is similarly used in combination with the tissue clearing method based on a CUBIC reagent, and an image obtained by turning the micrograph into a three-dimensional model are shown in FIG. **4B****.**

Experimental procedures used in Test Examples A to H are described below.

### <<Fluorescent Staining of Tissue Section>>

A tissue to be stained was washed with phosphate buffered saline (PBS), was immersed in 30% (w/v) of sucrose in the PBS, and was frozen in O.C.T. COMPOUND (45833, Sakura Finetek) at - 80°C overnight. The frozen tissue was cut into a thickness of 10 um with a cryostat (CM3050 S, Leica Biosystems). The resultant frozen section was washed with PBS three times, and was subjected to pretreatment with a 0.5% solution of periodic acid (HIO₄) (86171, MUTO PURE CHEMICALS CO., LTD.) at room temperature for 30 minutes. The section was washed with PBS three times, and was incubated in FAM hydrazide, 5-isomer (50 µM, 24170, BroadPharm, in this description, sometimes referred to as "FAM hydrazide"), Alexa Fluor 488 hydrazide (50 µM, A10436, ThermoFisher Scientific, Inc.), BDP FL hydrazide (50 µM, 11470, Lumiprobe Corporation), or fluorescein (50 µM, FUJIFILM Wako Pure Chemical Corporation) diluted with PBS at room temperature overnight. After that, the section was washed with PBS and an image thereof was obtained with a confocal microscope.

### <<Whole-mount 3D Tissue Staining (Combined Use of Tissue Clearing and Fluorescent Staining)>>

CUBIC-L and CUBIC-R+ reagents (T3740 and T3741, Tokyo Chemical Industry Co., Ltd.) were used in tissue clearing. A tissue removal procedure is simply described below.

A formaldehyde-fixed tissue specimen was washed with PBS, and was subsequently immersed in a 50% (v/v) CUBIC-L reagent (mixture containing water and the CUBIC-L reagent at 1:1) overnight. Further, the specimen was immersed in the CUBIC-L reagent at 37°C for 5 days or 6 days while being gently shaken. Next, each of those specimens was washed with PBS, was immersed in 30% (w/v) of sucrose in the PBS, and was frozen in O.C.T. COMPOUND at -80°C overnight. Next, the frozen specimen was thawed, was washed with PBS, and was immersed in a 0.5% solution of periodic acid (HIO₄) (86171, MUTO PURE CHEMICALS CO., LTD.) at room temperature for 30 minutes. After having been washed with PBS, the specimen was stained by being gently shaken at room temperature for 2 days or 3 days together with FAM hydrazide, 5-isomer (50 µM), Compound 1 (50 µM), Alexa Fluor 488 hydrazide (50 µM), BDP FL hydrazide (50 µM), or fluorescein (50 µM) in PBS containing 0.5% (v/v) of Triton X-100 (12967, Nacalai Tesque, Inc.). When nuclear counterstaining was performed, propidium iodide (PI) (10 µg/mL, P21493, Life Technologies Corporation) or RedDot-2 (1:100, 40061-T, Biotium, Inc.) was also added to the staining solution. After the staining step, the specimen was washed with PBS, and was immersed in a 50% (v/v) CUBIC-R+ reagent (mixture containing water and the CUBIC-R+ reagent at 1:1) overnight. Further, the specimen was immersed in the CUBIC-R+ reagent for 1 day or 2 days while being gently shaken. A cleared tissue specimen thus obtained was subjected to 3D imaging with a confocal fluorescence microscope or a light-sheet fluorescence microscope. A wavelength of 488 nm was applied as the excitation wavelength of each of FAM hydrazide, Compound 1, Alexa Fluor 488 hydrazide, BDP FL hydrazide, and fluorescein, and the fluorescence detection wavelength range of each of these materials was from 490 nm to 562 nm.

### <<Microscopy and Image Analysis>>

A 3D image was obtained by using a confocal microscope (Zeiss LSM880 Confocal/Multiphoton, Carl Zeiss AG) or a light-sheet fluorescence microscope (Zeiss Lightsheet 7, Carl Zeiss AG). All pieces of raw image data were reconstructed and analyzed with Imaris software (version 9.2.1, Bitplane Inc.).

### <<Statistical Analysis>>

The normality of two sets of data was evaluated by a Kolmogorov and Smirnov test at a significance level of 0.05. When the normality was recognized in the groups, the homogeneity of variance was tested by an F-test at a significance level of 0.05. When the two groups followed a normal distribution with equal variance, a Student's t-test was applied, and when the two groups followed a normal distribution without equal variance, a Welch's t-test was applied. When no normality was recognized in the groups, a Mann-Whitney U-test was used.

### [Test Example A] Fluorescent Staining of Frozen Sliced Specimen of Human Colonic Mucosa

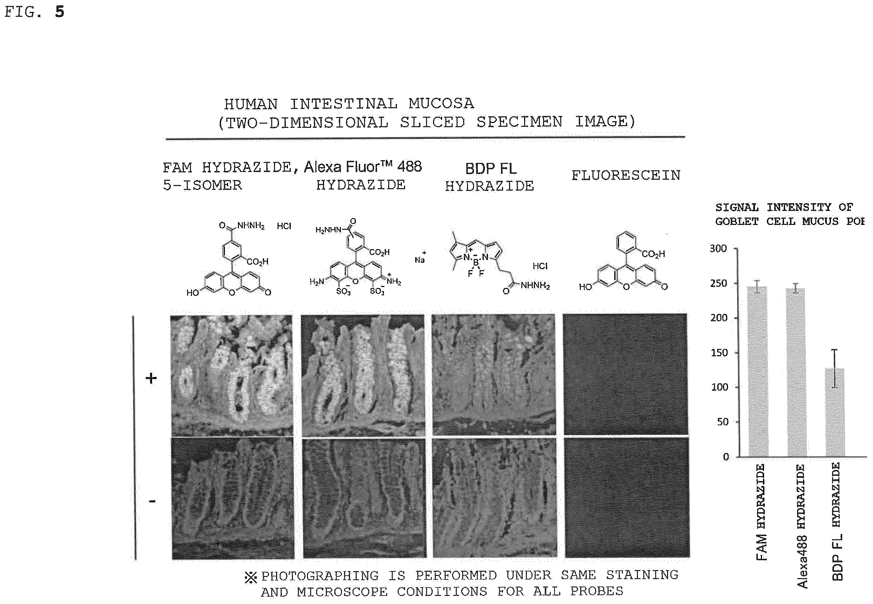

A section of a human colorectal mucosal tissue sampled from the non-tumor region of a surgical specimen of colorectal cancer was subjected to fluorescent staining through use of FAM hydrazide, Alexa Fluor 488 hydrazide, BDP FL hydrazide, or fluorescein serving as a fluorescent skeleton as a stain. The results are shown in FIG. **5****.**

As shown in FIG. **5****,** a clear positive image in goblet cell mucus was obtained in each of FAM hydrazide and Alexa Fluor 488 hydrazide. A reaction between an aldehyde group, and the NH₂ group of each of FAM hydrazide and Alexa Fluor 488 hydrazide was conceived as a staining principle because those signals were not obtained when oxidation treatment with HIO₄ was not performed. In addition, in BDP FL hydrazide, the obtained signal was weak, and in fluorescein, no fluorescence was observed.

### [Test Example B] Clearing and Fluorescent Staining of Human Colonic Mucosal Tissue

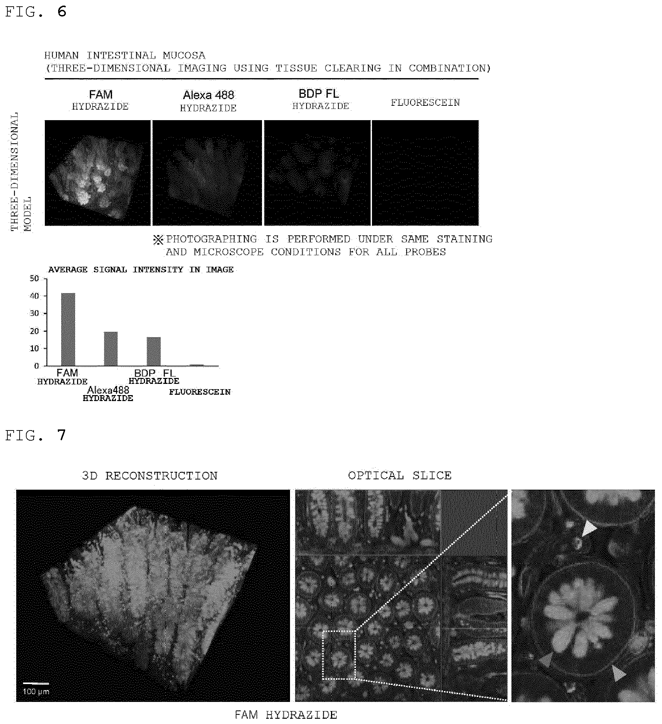

A human colonic mucosal tissue after its formalin fixation, which had been excised into a square about 4 mm on a side, was subjected to clearing treatment and to whole-mount 3D tissue staining through use of FAM hydrazide, Alexa Fluor 488 hydrazide, BDP FL hydrazide, or fluorescein as a stain. The results are shown in FIG. **6** to FIG. **9****.**

As shown in FIG. **6****,** in the 3D imaging, a clear stereoscopic image was obtained in FAM hydrazide. Meanwhile, in Alexa Fluor 488 hydrazide and BDP FL hydrazide, the obtained signals were weak, and in fluorescein, no fluorescence was observed.

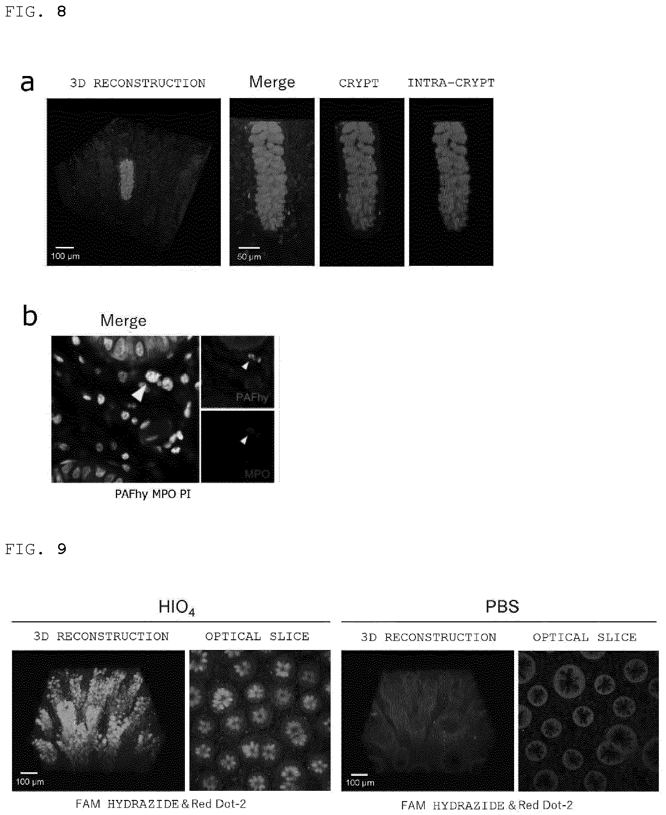

Meanwhile, when FAM hydrazide was used, a goblet cell and a basement membrane in a crypt of a colonic mucosa were clearly stained, and hence the stereoscopic structure of the crypt was able to be imaged in an extremely clear manner (FIG. 7). In addition, combined use of the staining and three-dimensional image analysis enabled the performance of imaging at a single-crypt level (FIG. **8a**). It is understood from the observation that in a normal colonic crypt, mucus incorporated into goblet cells is uniform, and hence the cells are regularly arrayed. A neutrophil, which was also a PAS staining-positive cell, was clearly stained even in the 3D tissue staining with FAM hydrazide, and the fact was recognized by immunohistostaining with an anti-myeloperoxidase (MPO) antibody (FIG. **8b****,** in the figure, the term "PAFhy" represents FAM hydrazide). In addition, when oxidation treatment with HIO₄ was not performed, no positive signal was observed. Accordingly, even in a 3D imaging method including using a CUBIC reagent, the bonding of the NH₂ group of FAM hydrazide to an aldehyde group produced by oxidation was conceived as a staining principle (FIG. **9**).

### [Test Example C] 3D Imaging of Histopathological Analyte

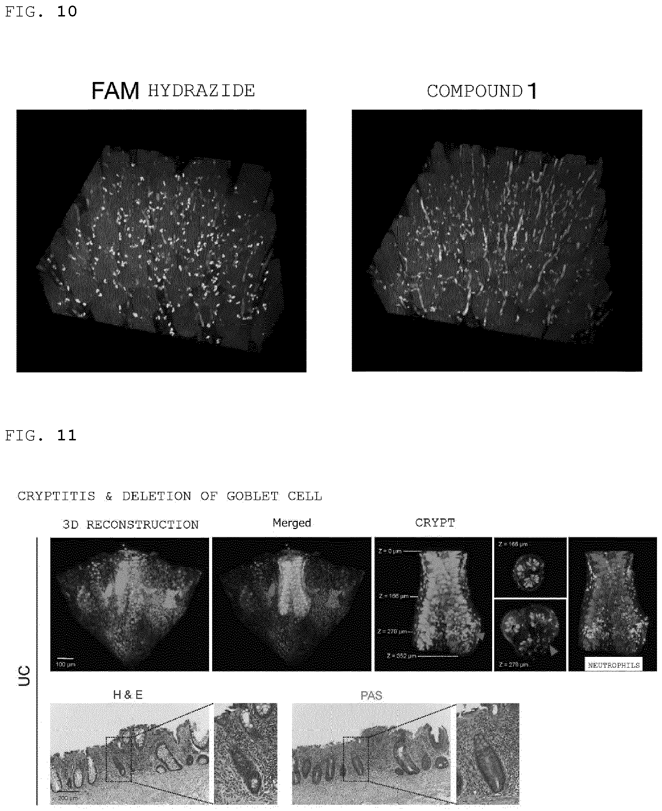

A colonic tissue analyte collected from an individual suffering from ulcerative colitis was subjected to clearing treatment and to whole-mount 3D tissue staining through use of FAM hydrazide or Compound 1 as a stain. The results are shown in FIG. **10****.**

As shown in FIG. **10****,** in 3D imaging, Compound 1 was able to provide a clear stained image of a neutrophil as in FAM hydrazide.

Each of the stains (DAF-2, Compound 2, Compound 1, FAM hydrazide, Alexa Fluor 488 hydrazide, and BDP FL hydrazide) used in Examples and Test Examples described above has an excitation wavelength in the range of from 400 nm to 520 nm, and hence generates green fluorescence. Alexa Fluor 488 hydrazide is typically a first choice as a green fluorescent dye because of its high fluorescence quantum yield and high light stability, and in actuality, the use of Alexa Fluor 488 hydrazide enabled clear staining of a sliced section. Meanwhile, with regard to the staining of a sample having a thickness after clearing treatment, it was found that when Alexa Fluor 488 hydrazide and BDP FL hydrazide were used, staining properties were insufficient, and the staining property of a compound having a fluorescein skeleton (DAF-2, Compound 2, Compound 1, or FAM hydrazide) was significantly excellent. Although the reason why such effect is exhibited is unclear, assumed reasons are, for example, as follows: those compounds are each excellent in tissue permeability; and the compounds are each hardly affected by a clearing agent.

### [Test Example D] 3D Imaging of Histopathological Analyte

A colonic tissue analyte collected from an individual suffering from ulcerative colitis was subjected to clearing treatment and to whole-mount 3D tissue staining through use of FAM hydrazide as a stain. In addition, glass slides for classical HE staining and PAS staining were also produced from a tissue continuous with the analyte to be subjected to those kinds of treatment. The results are shown in FIG. **11** to FIG. **13****.**

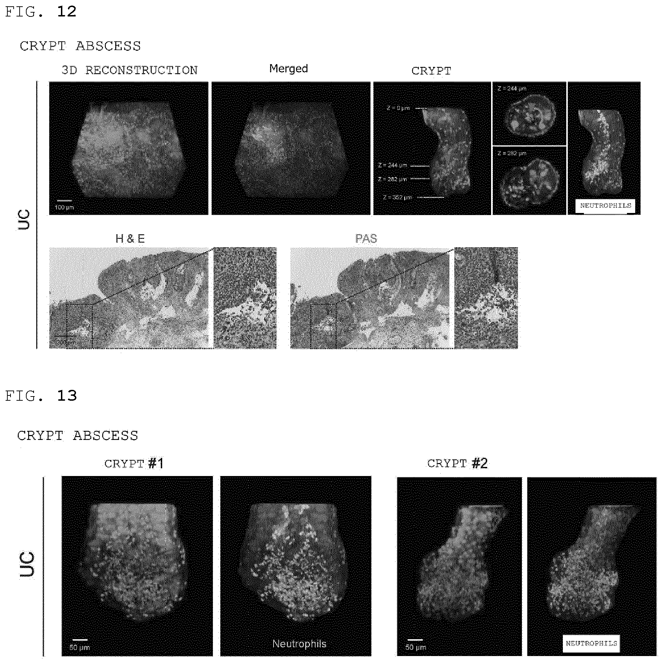

As shown in FIG. **11****,** in an example involving a high degree of inflammatory cell infiltration, the finding of cryptitis, that is, neutrophils (indicated by ▲) infiltrating into a crypt, and a finding in which the mucus of a goblet cell was reduced by inflammation (goblet cell depletion) were clearly imaged. Next, attention was paid to the finding of a crypt abscess characteristic of ulcerative colitis, and as a result, the finding of a crypt abscess was observed in the same case (FIG. **12**). In the finding, the following was found: minute abscesses were formed at the bottom; and these abscesses were formed by the neutrophils (indicated by A) that had infiltrated from a diagonally downward direction of the crypt, and the neutrophils were distributed upward along a crypt lumen. In addition, even in the imaging of any other crypt abscess, an abscess portion was dominant at the bottom of a crypt, and a finding in which a crypt was broken in a substantially complete manner, and hence a boundary with its surroundings became unclear was observed (FIG. **13**). It was found from the foregoing that a 3D imaging method including using FAM hydrazide enabled the three-dimensional observation of the pathological crypt structure of ulcerative colitis, and enabled the stereoscopic distribution pattern of neutrophils that was impossible to evaluate two-dimensionally.

### [Test Example E] First Example of 3D Imaging of Pathological Finding of Inflammatory Bowel Disease and Pathological Diagnosis based on the 3D Imaging

Tissue fragments were cut out of the surgical materials of ulcerative colitis (UC), Crohn's disease (CD), and non-specific colitis (Non-IBD: a non-tumor portion in a colon cancer analyte), and a half of the fragments were subjected to clearing treatment and to whole-mount 3D tissue staining through use of FAM hydrazide as a stain. In addition, glass slides for classical HE staining and PAS staining were produced by using the other half thereof.

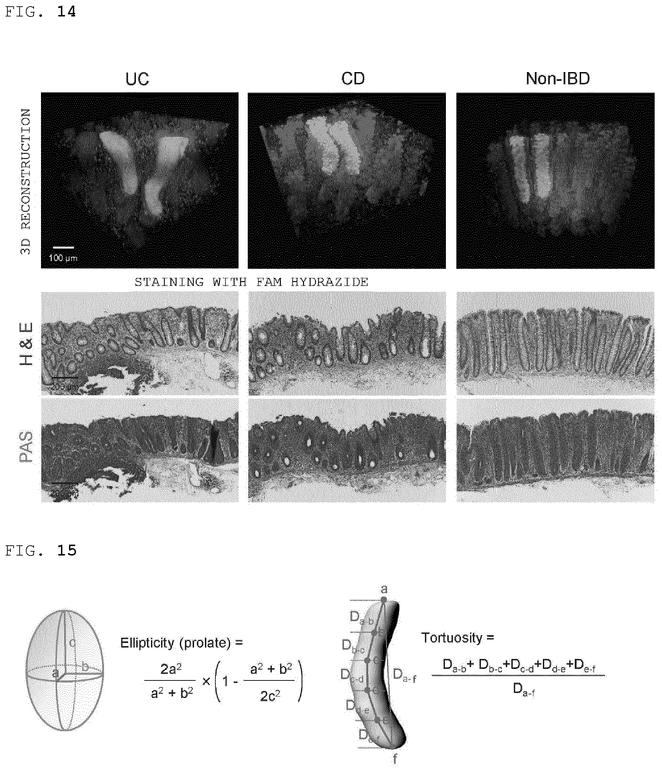

As shown in FIG. **14****,** according to the 3D tissue staining, crypt distortion considered to be one finding of ulcerative colitis was able to be three-dimensionally imaged. The distortion was frequently observed in ulcerative colitis, and moreover, was also observed in Crohn's disease.

Next, the possibility that an inflammatory bowel disease that was difficult to diagnose based on a two-dimensional image was diagnosed based on the three-dimensional structure of a crypt was investigated. Specifically, attention was paid to the degree of crypt distortion, and to more quantitatively evaluate the distortion, three parameters, that is, a volume, an ellipticity (prolate), and a tortuosity were defined in the three-dimensional analysis of the crypt, followed by the multidisciplinary observation and quantification of these parameters for each of the analytes of ulcerative colitis, Crohn's disease, and non-specific colitis. The volume was determined by processing a 3D image of the crypt with image analysis software (Imaris). The ellipticity was calculated from an equation illustrated in FIG. **15** by elliptically approximating the 3D image of the crypt. In addition, the tortuosity was calculated as follows: points "a" to "f" were set in the central portions of the crypt in optical slices at respective depths when the crypt was divided into 5 sections; the distance (Da-f) of a straight line connecting the upper end (point "a") and the lower end (point "f"), and the total sum of distances (Da-b, Db-c, Dc-d, Dd-e, and De-f) between adjacent points out of the points "a" to "f" were calculated; and the tortuosity was calculated on the basis of these values.

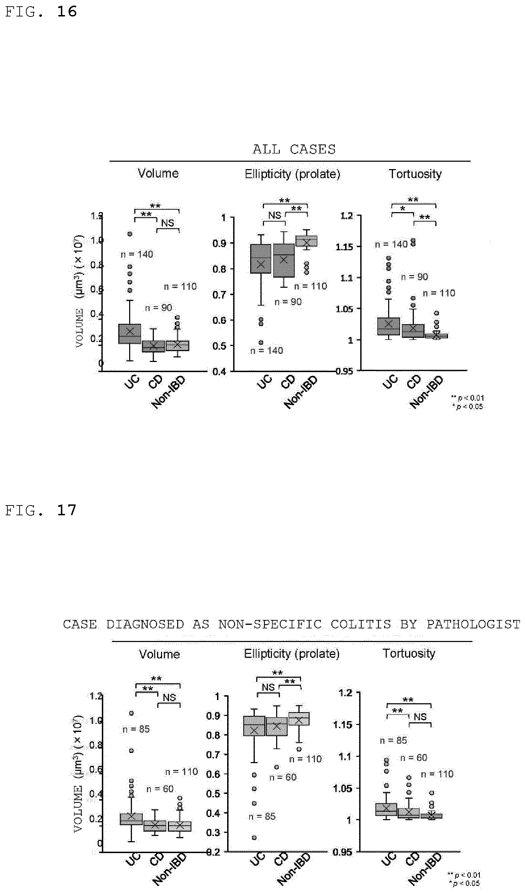

As a result of the analysis through use of all the cases, statistically significant differences were obtained in all the parameters, that is, the volume, the ellipticity, and the tortuosity (FIG. **16**). Specifically, the volume increased in ulcerative colitis as compared to those in the other two cases, and a reduction in value of the ellipticity was observed in each of ulcerative colitis and Crohn's disease as compared to that in non-specific colitis. The following difference in value of the tortuosity was observed: the tortuosity reduced in the order of ulcerative colitis, Crohn's disease, and non-specific colitis. In addition, even when the analysis was performed only in a case diagnosed as non-specific colitis by a pathologist, substantially the same results were obtained, and statistically significant differences were observed in the three parameters (FIG. **17**). The foregoing suggests that the three-dimensional quantitative values with respect to the crypt distortion enable the diagnosis of an inflammatory bowel disease that is difficult to diagnose by a two-dimensional and classical pathological diagnosis method.

In the conventional histopathological diagnosis of a disease, such as ulcerative colitis or Crohn's disease, the presence or absence of a characteristic finding, such as a crypt abscess or epithelioid granuloma, generally provides a useful clue. However, in a case in which inflammation is not manifested by therapeutic modification or the like, such finding is not obtained in some cases. In such cases, it can only be said histopathologically that the case is non-specific colitis. Accordingly, in actuality, it is difficult to definitely diagnose the case only from histopathological diagnosis into which no clinical information is incorporated. In fact, when glass slides for the HE staining and PAS staining of ulcerative colitis, Crohn's disease, and non-specific colitis were diagnosed by pathologists, 58.6% of the glass slides used for ulcerative colitis and 63.1% of the glass slides used for Crohn's disease were diagnosed as non-specific colitis, and the results of diagnosis did not coincide between the pathologists for 31.0% of the glass slides used for ulcerative colitis and 26.3% of the glass slides used for Crohn's disease. Those tissue analytes were collected from sites having different color tones, and there was no significant difference in degree of inflammation between the analytes.

### [Test Example F] Second Example of 3D Imaging of Pathological Finding of Inflammatory Bowel Disease and Pathological Diagnosis based on the 3D Imaging

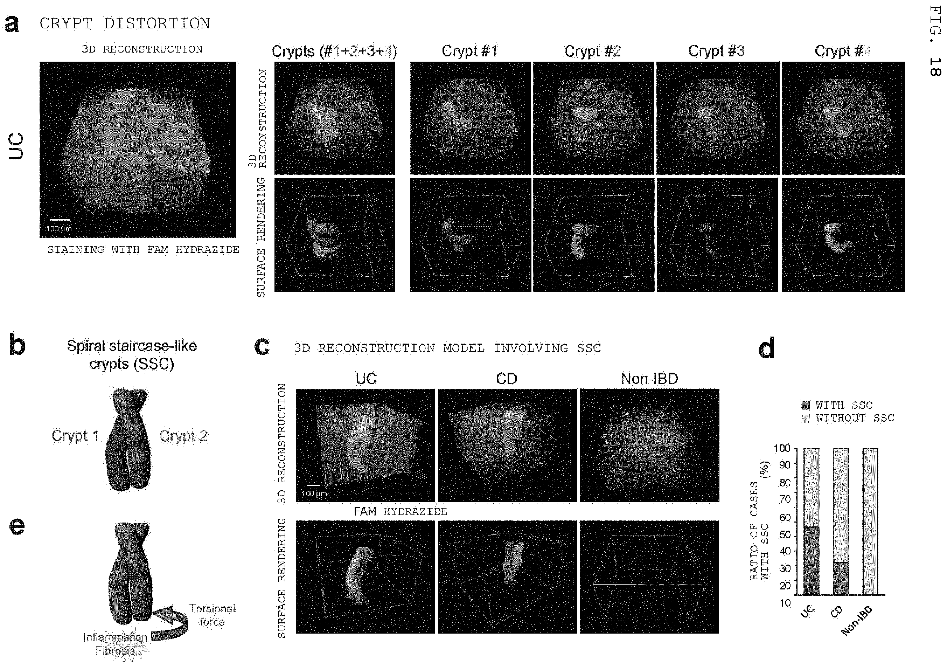

With regard to each of the 3D images obtained in Test Example E, more detailed three-dimensional morphological observation of the crypt distortion was performed. As a result, it was found that in ulcerative colitis, a distorted structure of such a shape that a plurality of crypts were involved was present (FIG. **18a**). In the structure, all of the plurality of crypts were distorted in the same direction. Such a three-dimensional finding in which two or more adjacent crypts were distorted in the same direction was defined as "spiral staircase-like crypts (SSCs)" (FIG. **18b**), and the number of the findings was counted.

As a result, the finding was observed in 46.4% of cases diagnosed as ulcerative colitis, and its frequency was higher than that in Crohn's disease, that is, 22.2% (FIG. **18c** and FIG. **18d**). In addition, the finding was not observed in non-specific colitis. Those results suggest that the presence or absence of a finding peculiar to an inflammatory bowel disease enables the diagnosis of the inflammatory bowel disease that is difficult to diagnose by a two-dimensional and classical pathological diagnosis method. An assumed factor for the formation of the finding was as follows (FIG. **18e**): the same region in which a plurality of adjacent crypts were involved was distorted as a whole probably by a pressure difference due to inflammation or fibrosis.

### [Test Example G] 3D Imaging of Human Colonic Mucosal Tissue

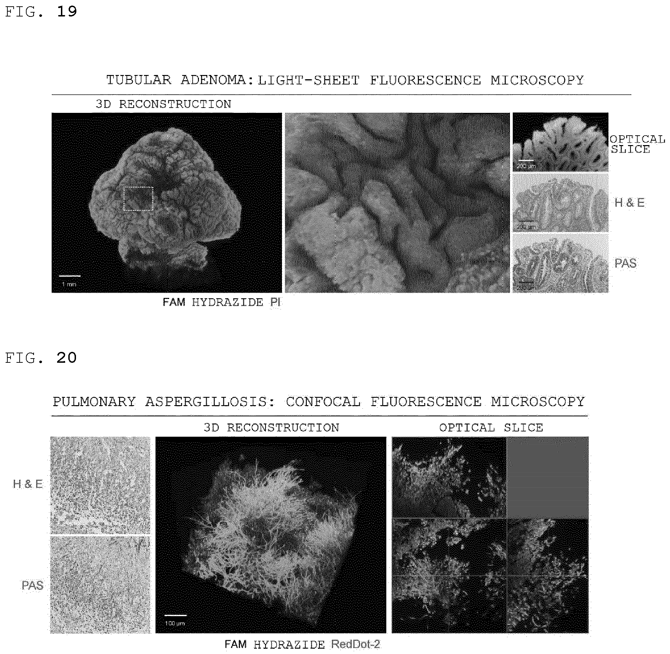

The entirety of a human colonic polyp was subjected to 3D tissue staining through use of each of FAM hydrazide and propidium iodide (PI) as a stain, and its observation and 3D imaging were performed with a light-sheet microscope. The results are shown in FIG. **19** together with images obtained by HE staining and PAS staining.

As shown in FIG. **19****,** the intricate and protuberant structure of the surface layer of the human colonic polyp was able to be observed.

### [Test Example H] 3D Imaging of Fungal Hyphae

Fungal hyphae in a pulmonary aspergillosis tissue were subjected to 3D tissue staining through use of each of FAM hydrazide and RedDot (trademark) 2 as a stain, and their observation and 3D imaging were performed with a confocal microscope. The results are shown in FIG. **20** together with images obtained by HE staining and PAS staining.

As shown in FIG. **20****,** the structures of the fungal hyphae were able to be clearly visualized.

### Industrial Applicability

The fluorescent staining method of the present invention can contribute to the identification of, for example, a lesion in the stereoscopic structure of an intestinal crypt because the method enables suitable detection of a substance containing a glycol group in a tissue or cell sample that has a three-dimensional stereoscopic structure and is subjected to clearing treatment.

## Claims

1. A method of fluorescently staining a substance containing a glycol group in a tissue or cell sample, the method comprising bringing a compound represented by the following general formula (I) or a salt thereof and the tissue or cell sample into contact with each other: 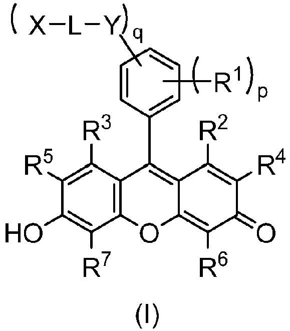 in the general formula (I):
R¹ represents a carboxyl group, an alkyl group that may be substituted, an alkoxy group that may be substituted, or a halogen atom;
R², R³, R⁴, R⁵, R⁶, and R⁷ each independently represent a hydrogen atom, a hydroxyl group (OH group), a halogen atom, or a linear or branched alkyl group that may be substituted;
X represents a hydrazide group, a semicarbazide group, a thiosemicarbazide group, a hydrazino group, a hydroxylamino group, an amino group, or an alkylamino group;
Y is absent or represents an amide group, an ester group, a carbamide group, a thiourea group, a carbamate group, a carbonate group, or an oxygen atom;
L represents -(linear or branched alkylene)ₙ- where "n" represents an integer of from 1 to 10, -(linear or branched alkylene-O-)ₙ- where "n" represents an integer of from 1 to 10, or -(linear or branched alkylene-O-)ₙ-(linear alkylene)ₘ- where "n" and "m" each independently represent an integer of from 1 to 10, or represents a single bond;
"p" represents an integer of from 0 to 4, and when "p" represents 2 or more, R¹s may be identical to or different from each other; and
"q" represents an integer of 1 or 2, and when "q" represents 2, structures each represented by (X-L-Y) may be identical to or different from each other, and a sum of "p" and "q" is an integer of 5 or less.

2. The method according to claim 1, further comprising oxidizing a glycol group in the tissue or cell sample to produce an aldehyde group before bringing the compound or the salt thereof and the tissue or cell sample into contact with each other.

3. The method according to claim 1, wherein the tissue or cell sample has a three-dimensional stereoscopic structure.

4. The method according to claim 1, wherein the tissue or cell sample is a tissue or cell sample subjected to clearing treatment.

5. The method according to claim 1, further comprising treating the tissue or cell sample with a hydrophilic clearing agent to clear the tissue or cell sample.

6. A method of detecting a substance containing a glycol group in a tissue or cell sample, the method comprising:
subjecting the substance containing a glycol group in the tissue or cell sample to fluorescent staining by the method of any one of claims 1 to 5; and
measuring fluorescence derived from the compound.

7. A method of visualizing a tissue or a cell containing a substance containing a glycol group, the method comprising:
subjecting the substance containing a glycol group in a tissue or cell sample to fluorescent staining by the method of any one of claims 1 to 5; and
measuring and imaging fluorescence derived from the compound.

8. The method according to claim 7, wherein the tissue or the cell containing the substance containing a glycol group is a mucosal epithelium, a basement membrane, or a fungus.

9. The method according to claim 7, wherein the method is a method of visualizing a crypt.

10. A method of determining a tissue and/or a lesion containing a large amount of a substance containing a glycol group in a tissue or cell sample, the method comprising:
subjecting the substance containing a glycol group in the tissue or cell sample to fluorescent staining by the method of any one of claims 1 to 5; and
determining that the tissue and/or the lesion is present in a stained site.

11. The method according to claim 10, wherein the lesion is crypt distortion.

12. A method of screening a therapeutic agent for a disease involving a lesion of a tissue or a cell containing a substance containing a glycol group, the method comprising:
administering a therapeutic agent candidate to an individual suffering from the disease;
collecting a tissue or cell sample in which the lesion occurs from the individual;
subjecting the substance containing a glycol group in the tissue or cell sample to fluorescent staining by the method of any one of claims 1 to 5;
measuring and imaging fluorescence derived from the compound to visualize the tissue or the cell containing the substance containing a glycol group; and
determining a therapeutic effect of the therapeutic agent candidate on the basis of an image of the tissue or the cell containing the substance containing a glycol group that has been visualized.

13. The method according to claim 12, wherein the disease is an inflammatory bowel disease.

14. A composition for fluorescent staining, comprising a compound represented by the following general formula (I) or a salt thereof, wherein the composition is used for visualizing a tissue or a cell containing a substance containing a glycol group: 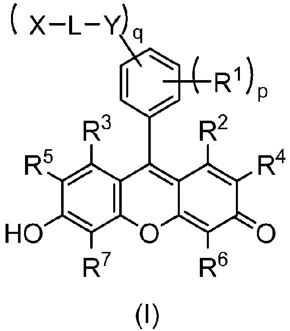 in the general formula (I):
R¹ represents a carboxyl group, an alkyl group that may be substituted, an alkoxy group that may be substituted, or a halogen atom;
R², R³, R⁴, R⁵, R⁶, and R⁷ each independently represent a hydrogen atom, a hydroxyl group (OH group), a halogen atom, or a linear or branched alkyl group that may be substituted;
X represents a hydrazide group, a semicarbazide group, a thiosemicarbazide group, a hydrazino group, a hydroxylamino group, an amino group, or an alkylamino group;
Y is absent or represents an amide group, an ester group, a carbamide group, a thiourea group, a carbamate group, a carbonate group, or an oxygen atom;
L represents -(linear or branched alkylene)ₙ- where "n" represents an integer of from 1 to 10, -(linear or branched alkylene-O-)ₙ- where "n" represents an integer of from 1 to 10, or -(linear or branched alkylene-O-)ₙ-(linear alkylene)ₘ- where "n" and "m" each independently represent an integer of from 1 to 10, or represents a single bond;
"p" represents an integer of from 0 to 4, and when "p" represents 2 or more, R¹s may be identical to or different from each other; and
"q" represents an integer of 1 or 2, and when "q" represents 2, structures each represented by (X-L-Y) may be identical to or different from each other, and a sum of "p" and "q" is an integer of 5 or less.

15. The composition for fluorescent staining according to claim 14, wherein the composition is used for visualizing a crypt.

16. The composition for fluorescent staining according to claim 14, further comprising a hydrophilic clearing agent.

17. A diagnostic composition for a disease involving a lesion of a tissue or a cell containing a substance containing a glycol group, comprising a compound represented by the following general formula (I) or a salt thereof: 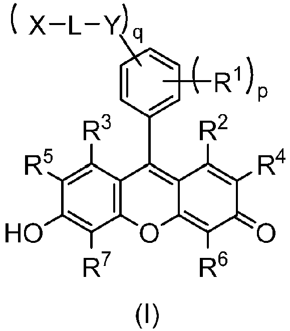 in the general formula (I):
R¹ represents a carboxyl group, an alkyl group that may be substituted, an alkoxy group that may be substituted, or a halogen atom;
R², R³, R⁴, R⁵, R⁶, and R⁷ each independently represent a hydrogen atom, a hydroxyl group (OH group), a halogen atom, or a linear or branched alkyl group that may be substituted;
X represents a hydrazide group, a semicarbazide group, a thiosemicarbazide group, a hydrazino group, a hydroxylamino group, an amino group, or an alkylamino group;
Y is absent or represents an amide group, an ester group, a carbamide group, a thiourea group, a carbamate group, a carbonate group, or an oxygen atom;
L represents -(linear or branched alkylene)ₙ- where "n" represents an integer of from 1 to 10, -(linear or branched alkylene-O-)ₙ- where "n" represents an integer of from 1 to 10, or -(linear or branched alkylene-O-)ₙ-(linear alkylene)ₘ- where "n" and "m" each independently represent an integer of from 1 to 10, or represents a single bond;
"p" represents an integer of from 0 to 4, and when "p" represents 2 or more, R¹s may be identical to or different from each other; and
"q" represents an integer of 1 or 2, and when "q" represents 2, structures each represented by (X-L-Y) may be identical to or different from each other, and a sum of "p" and "q" is an integer of 5 or less.

18. The diagnostic composition according to claim 17, wherein the disease is an inflammatory bowel disease.

19. The diagnostic composition according to claim 17, further comprising a hydrophilic clearing agent.

20. A compound represented by the following general formula (I) or a salt thereof: 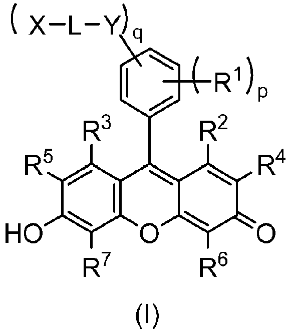 in the general formula (I):
R¹ represents a carboxyl group, an alkyl group that may be substituted, an alkoxy group that may be substituted, or a halogen atom;
R², R³, R⁶, and R⁷ each independently represent a hydrogen atom, a hydroxyl group (OH group), a halogen atom, or a linear or branched alkyl group that may be substituted;
R⁴ and R⁵ each represent a fluorine atom;
X represents a hydrazide group, a semicarbazide group, a thiosemicarbazide group, a hydrazino group, or a hydroxylamino group;
Y is absent or represents an amide group, an ester group, a carbamide group, a thiourea group, a carbamate group, a carbonate group, or an oxygen atom;
L represents -(linear or branched alkylene)ₙ- where "n" represents an integer of from 1 to 10, -(linear or branched alkylene-O-)ₙ- where "n" represents an integer of from 1 to 10, or -(linear or branched alkylene-O-)ₙ-(linear alkylene)ₘ- where "n" and "m" each independently represent an integer of from 1 to 10, or represents a single bond;
"p" represents an integer of from 0 to 4, and when "p" represents 2 or more, R¹s may be identical to or different from each other; and
"q" represents an integer of 1 or 2, and when "q" represents 2, structures each represented by (X-L-Y) may be identical to or different from each other, and a sum of "p" and "q" is an integer of 5 or less.

21. A compound represented by the following general formula (I) or a salt thereof: 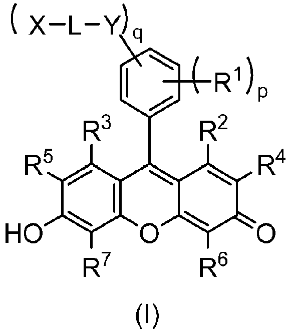 in the general formula (I):
R¹ represents an alkyl group that may be substituted, an alkoxy group that may be substituted, or a halogen atom;
R², R³, R⁴, R⁵, R⁶, and R⁷ each independently represent a hydrogen atom, a hydroxyl group (OH group), a halogen atom, or a linear or branched alkyl group that may be substituted;
X represents an amino group or an alkylamino group;
Y is absent;
L represents a single bond;
"p" represents an integer of from 0 to 4, and when "p" represents 2 or more, R¹s may be identical to or different from each other; and
"q" represents an integer of 1 or 2, and when "q" represents 2, structures each represented by (X-L-Y) may be identical to or different from each other, and a sum of "p" and "q" is an integer of 5 or less.
